# EUROPEAN PATENT SPECIFICATION

(11) **EP 2 139 889 B1**
(45) Date of publication and mention of the grant of the patent: **19.12.2012**
(21) Application number: 08716612.0
(22) Date of filing: 18.03.2008
(51) Int. Cl.: C07D 471/04, A61K 31/4375, A61P 31/12

(54) **AMINO-NAPHTHYRIDINE DERIVATIVES**
AMINONAPHTHYRIDINDERIVATE
DÉRIVÉS AMINO-NAPHTHYRIDINE

(30) Priority: 19.03.2007 EP 07005561
(43) Date of publication of application: 06.01.2010
(73) Proprietor: Technische Universität Carolo-Wilhelmina zu Braunschweig, 38106 Braunschweig (DE)
(72) Inventor: MAZIK, Monika, 38106 Braunschweig (DE)
(74) Representative: Kröncke, Rolf
(86) International application number: PCT/EP2008/002160
(87) International publication number: WO 2008/113558

(56) References cited:
- EP-A- 1 714 966
- WO-A-00/50424
- WO-A-01/70742
- WO-A-2007/022946
- US-A1- 2004 044 207
- M. WOZNIAK ET. AL.: "Reactions of 2-Chloro-1,8-naphthyridine with Some Nucleophiles." POLISH JOURNAL OF CHEMISTRY, vol. 55, 1981, pages 2427-2437, XP009088819
- A. MANGINI ET. AL.: "Contributo alla conoscena delle naftiridine. Nota III. derivati della 2,4-dimethilnaftiridina." GAZZETTA CHIMICA ITALIANA, vol. 73, 1943, pages 323-329, XP009088817
- B. OSKUI ET. AL.: "Di- and trinuclear Pd(II) and Pt(II) complexes containing bridging 2,7-disubstituted naphthyridines." INORGANICA CHIMICA ACTA, vol. 287, 1999, pages 72-81, XP002448801

## Description

The present invention relates to new compounds having an amino-naphthyridine group. In particular, the present invention relates to new compounds, its stereoisomers and pharmaceutically acceptable salts or solvates thereof having a first unit (moiety) selected from the group of a phenyl derivative, a biphenyl derivative or a diphenyl alkane derivative and at least one amino-naphthyridine group linked with the first unit via a linking group. In specific embodiments, the present invention relates to compounds having a phenyl derivative unit and three amino-naphthyridine groups bound to the phenyl derivative unit via a linking group as well as salts or solvates thereof, in particular, pharmaceutically acceptable salts or solvates thereof. Further, the present invention relates to pharmaceutical compositions comprising said compounds. The compounds are particularly useful for treating or preventing infections, like viral infections.

### Background

Naphthyridine derivatives are known in the art and have been described to display various properties. For example, 1,8-naphthyridines have been described to function as CFR receptor antagonists, see EP 1 695 974. These naphthyridines acting as CFR receptor antagonists may represent novel antidepressant and/or anxiolytic drugs that may be useful in the treatment of the neuropsychiatric disorders manifested in hypersecretion of CRF. In US 2005/0182085 naphthyridine derivatives and physiologically tolerated salts and physiologically functional derivatives thereof are described which are beneficial in reducing blood glucose or having disturbances of lipid and carbohydrate metabolism, like the metabolic syndrome.

Further, anti-fungal, anti-viral and anti-bacterial properties of various naphthyridine derivatives are known. For example, in Bedard et al., Antimicrobialagents and Chemotherapy, April 2000, pages 929 to 937, the properties of a series of 1,6-naphthyridine and 7,8-diisochinoline derivatives exhibiting potent activity against human cytomegaloma virus have been described. Furthermore, naphthyridine derivatives having anti-viral activity are described in EP 1 714 966, WO 01/70742, US 2004/044207 and WO 00/50424, respectively.

WO-A-2007 022946 describes a number of N-benzyl-naphthyridineamine derivatives in which the benzyl moiety is substituted by alkoxy or halogen. Said compounds are useful for the treatment of neurodegenerative disorders.

WO-A-0050424 relates to N-acryl and N-benzyl - 1,8-naphthyridine-2-carboxy amides as antiviral compounds.

However, there is still a continuous need for compounds useful as active ingredients of pharmaceutically active and specific pharmaceuticals.

The present invention addresses the need for further compounds based on naphthyridine and its derivatives. That is, the present invention addresses to provide new effective and specific compounds based on naphthyridine and its derivatives. The invention provides new compounds composed of a so called spacer unit (first unit) and at least one naphthyridine unit. These molecules would be of beneficial use in a variety of applications, including treating or preventing infections, in particular, viral, bacterial or fungal infection. The present invention fulfils these and other needs.

### Summary of the invention

The present invention relates to the provision of new compounds composed of a spacer unit A and at least one amino-naphthyridine unit B bound to the spacer unit via a linker moiety, in particular, a methylene group as well as salts or solvates thereof, which are particularly useful in therapeutic or prophylactic treatment of various diseases, disorders or conditions. The present inventors recognized that the acyclic amino-naphthyridine based molecules are artificial receptors for carbohydrate recognition. In particular, the molecules of the present invention are able to bind selectively and transiently or permanently to e.g. N-acetylneuraminic acid (Neu5Ac). N-acetylneuraminic acid is known to be overexpressed on the cell surface of tumor cells and, in addition, sialic acids and derivatives, like Neu5Ac, are frequently used as a recognition site for viruses. Thus, in a further aspect, the present invention relates to pharmaceuticals containing the compounds of the present invention. These pharmaceuticals are particularly useful for preventing or treating infections, in particular, viral, parasitic, bacterial and fungal infections.

The newly developed compounds composed of the spacer unit and the amino-naphthyridine derivative units are able to interact specifically with neutral and ionic (anionic or cationic) sugar molecules like Neu5Ac which is a representative of an anionic sugar. The interaction involves hydrogen-bonding, CH-π-interactions, ion pairing and ionic hydrogen-bonding.

Thus, these new molecules are artificial receptors for the recognition of neutral and ionic sugar molecules, like Neu5Ac, allowing transient or permanent masking or blocking of sugar molecules, thus, preventing recognition and docketing of e.g. virus particles to host cells. That is, the new compounds may bind to its ligands present either on the surface of virus particles or other invaders, on host cells or present in the body fluid or extracellular space of an individual preventing interaction between said ligands and other molecules present on host cells, invaders, like virus, bacteria or parasites, the body fluid or the extracellular space.

Particular preferred, the amino-naphthyridine is a 2-amino-1,8-naphthyridine which may be substituted. In a preferred embodiment the spacer unit is a phenyl derivative of the general formula II.

The pharmaceutical composition comprising the compounds according to the present invention in effective amounts, optionally, together with pharmaceutically acceptable excipient can be used for the treatment or prevention of various diseases, disorders and conditions, in particular, infection diseases and cancer.

### Detailed description of the invention

The invention relates to compounds of the general formula I including stereoisomers and salts or solvates thereof

A-(CH₂-B)ₙ (I)

wherein
A is
i) a phenyl derivative of the general formula (II) 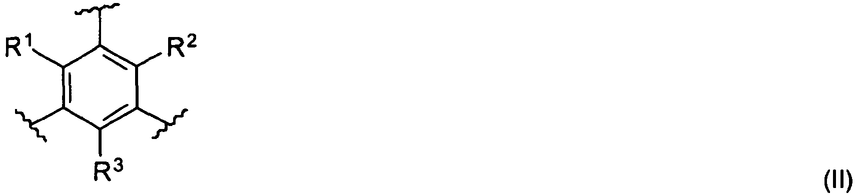 wherein each of R¹, R² and R³ being independently a C₁-C₆ alkyl group, a C₁-C₆ alkoxy group, a hydroxyl group or a halogen;
ii) a biphenyl derivative of the general formula (III) 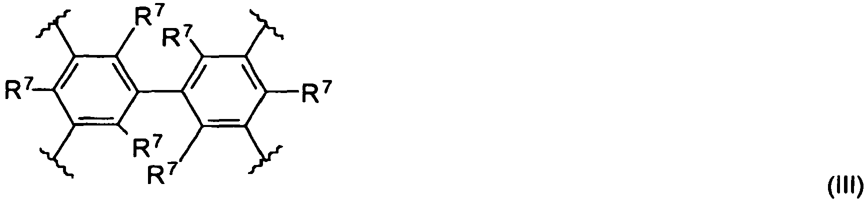 with each R⁷ being independently a hydrogen or a C₁-C₆ alkyl group, a C₁-C₆ alkoxy group, a hydroxyl group or a halogen;
iii) a diphenyl alkane derivative of the general formula (IV) 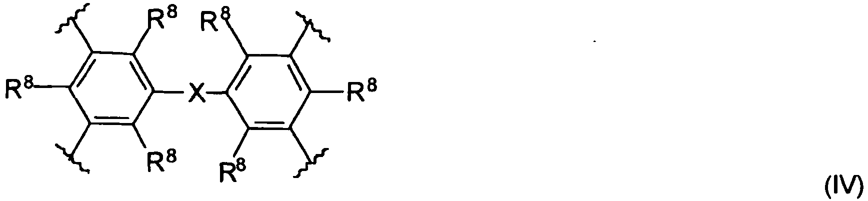 with each R⁸ being independently a hydrogen or a C₁-C₆ alkyl group, a C₁-C₆ alkoxy group, a hydroxyl group or a halogen;
   X is a C₁-C₆ alkyl group, in particular, a methylen group;
B is independently an amino-naphthyridine group of the general formula (V), (VI), or (VII) 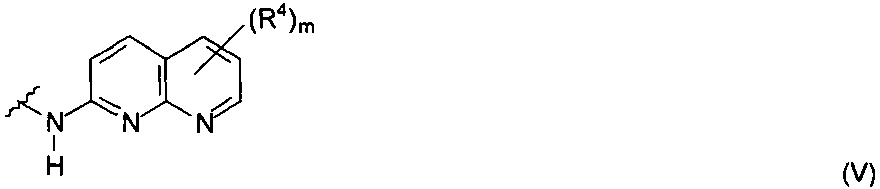 or 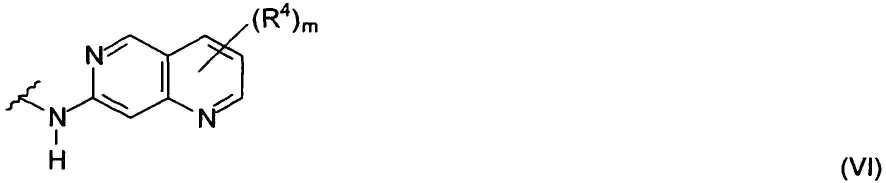 or 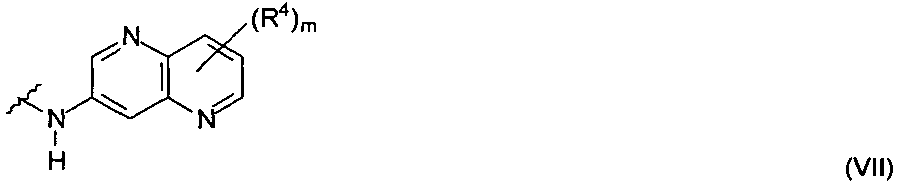 wherein each R⁴ is independently a hydrogen, a C₁-C₆ alkyl group, hydroxyl group, C₁-C₆ alkoxy group, halogen or NR¹⁰R¹¹ with R¹⁰ and R¹¹ being independently hydrogen or a C₁-C₆ alkyl group, and m is an integer of 2 to 5,
and n is an integer of 1, 2, or 3 if A is a phenyl derivative of formula (II) or n is 1, 2, 3, or 4 if A is (III) or (IV).

B may be identical or different from each other. Preferably, if n is ≥2 all B are identical.

A preferred embodiment of the present invention relates to compounds wherein B is independently an amino-naphthyridine group of the general formula (Va), (VIa), or (VIIa) 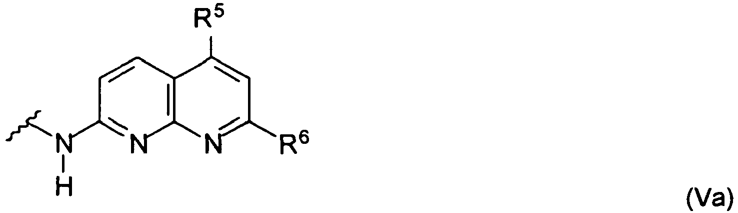 or 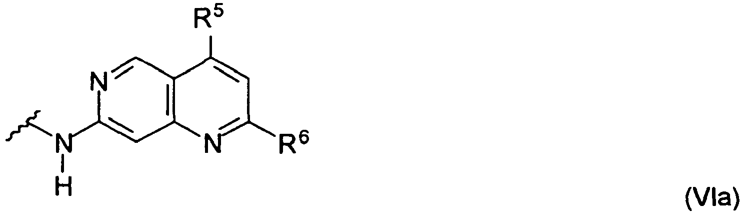 or 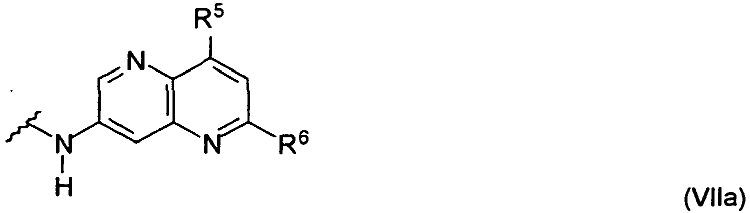 wherein R⁵ or R⁶ being independently a C₁-C₆ alkyl group, hydroxyl group, C₁-C₆ alkoxy group, halogen or NR¹⁰R¹¹ with R¹⁰ and R¹¹ being independently hydrogen or a C₁-C₆ alkyl group.

In a preferred embodiment the compound of the general formula I is a physiologically tolerated and pharmaceutically acceptable salt or solvate.

The invention relates to compounds of the formula I in the form of their racemates, racemic-mixtures and pure enantiomers and their diastereomers and mixtures thereof.

Pharmaceutically acceptable salts are particularly suitable for medical applications because their solubility in water is greater than that of the initial or basic compounds. Suitable pharmaceutically acceptable acid addition salts of the compounds of the invention are salts of inorganic acids such as hydrochloric acid, hydrobromic, phosphoric, metaphosphoric, nitric and sulphuric acid, and or organic acids, such as, for example, acetic acid, benzene-sulfonic, benzoic, citric, ethanesulfonic, fumaric, gluconic, glycolic, inothionic, lactic, lactobionic, maleic, malic, methanesulfonic, succinic, p-toluelenesulfonic and tartaric acid.

Within the scope of the present invention also salts with a pharmaceutically unacceptable anion are contemplated as well as the basic compounds. These salts with a pharmaceutically unacceptable anion may be useful as intermediates for the preparation or proliferation of pharmaceutically acceptable salts and/or for use in non-therapeutics, for example in vitro applications.

According to the present invention, the alkyl or alkoxy residues in the substituents R¹, R², R³, R⁴, R⁵, R⁶, R⁷, R⁸, R¹⁰, and R¹¹ may be both straight-chain and branched. Optionally, the alkyl or alkoxy residues may be substituted; in particular, substitution may be present with a hydroxyl group, halogen, nitro group or C₁-C₆ alkoxy group.

If residues or substituents may occur more than once in the compounds of the formula I, they may all, independently of one another, have the stated meanings and be identical or different.

In a preferred embodiment the spacer unit is a phenyl derivative of the general formula (II) 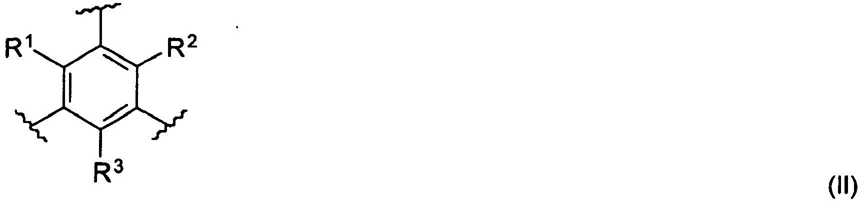 with each R¹, R² and R³ being a C₁-C₆ alkyl group, a C₁-C₆ alkoxy group, a hydroxyl group or a halogen. In a particular preferred embodiment the substituents R¹, R² and R³ are independently an alkyl group selected from the group of methyl, ethyl, propyl or butyl. In a more preferred embodiment substituents, R¹, R² and R³ are a methyl or ethyl group, preferably R¹, R² and R³ are identical.

In a particularly preferred embodiment the spacer unit is a phenyl derivative with R¹, R² and R³ being a methyl or ethyl group, the amino-naphthyridine unit is a 2-amino-1,8-naphthyridine unit with R⁶ is methyl and R⁵ is hydrogen. In another particularly preferred embodiment R¹, R², R³ are a methyl or ethyl group and R⁵ and R⁶ are a methyl group.

Another preferred embodiment relates to compounds wherein the spacer unit is a biphenyl derivative of the general formula (III) with each R⁷ being independently a hydrogen or C₁-C₆ alkyl group, a C₁-C₆ alkoxy group, a hydroxyl group or a halogen. Particularly preferred, the C₁-C₆ alkyl group is a methyl, ethyl, propyl or butyl group, whereby each R⁷ may have the same meaning or may be different. In particularly preferred embodiments, each R⁷ is independently hydrogen, methoxy, hydroxyl or methyl group. Specific embodiments of the compounds according to the present invention are compounds wherein each R⁷ is independently methyl or ethyl group and R⁶ is methyl, R⁵ is hydrogen or a methyl group and the amino-naphthyridine group is a 2-amino-1,8-naphthyridine.

In addition, in another embodiment of the present invention the spacer unit is a diphenyl alkane moiety of the general formula (IV) where the alkane X is a C₁-C₆ alkylene group, preferably a methylene or ethylene group. Said diphenyl alkane derivative may be substituted with substituents R⁸ where each R⁸ is independently hydrogen, C₁-C₆ alkyl group, C₁-C₆ alkoxy group, hydroxyl group or a halogen. Preferably, each R⁸ is independently a hydrogen, a methyl, ethyl, hydroxyl, methoxy or ethoxy group and, preferably, all R⁸ are identical.

That is, another preferred embodiment relates to a compound of the general formula I wherein A is a diphenyl methane derivative of the general formula (IV) wherein R⁸ is methyl, R⁶ of the 2-amino-1,8 naphthyridine moiety is methyl and R⁵ is hydrogen or methyl.

At least one amino-naphthyridine group of the general formulas (V), (VI), or (VII), in particular, of general formulas (Va), (VIa) or (VIIa) may be linked via the methylene group with the spacer molecule. That is, n is an integer of 1, 2 or 3 if the spacer moiety A is a phenyl derivative of formula (II) or n is an integer of 1, 2, 3, or 4 if A is a biphenyl derivative of general formula (III) or a diphenyl alkane derivative of general formula (IV).

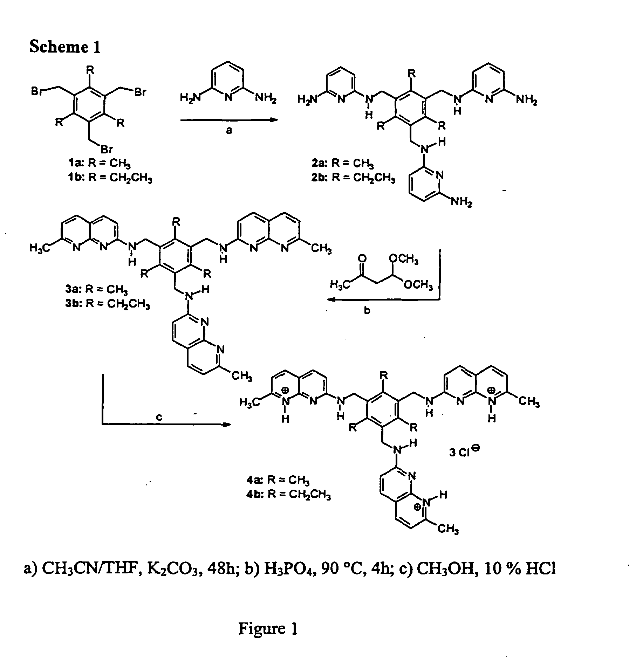

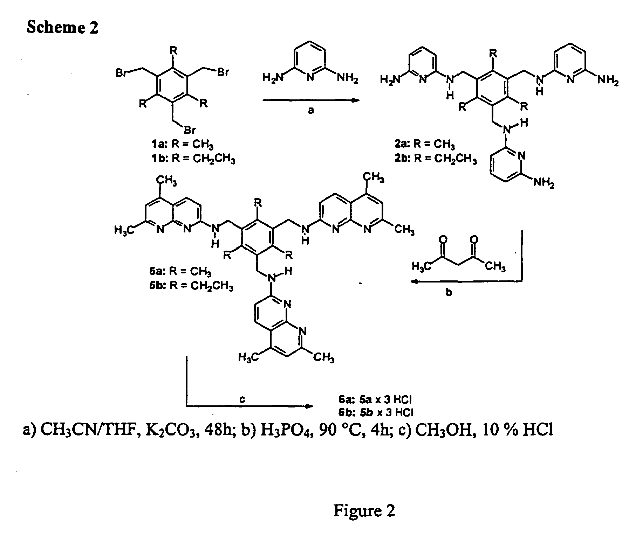

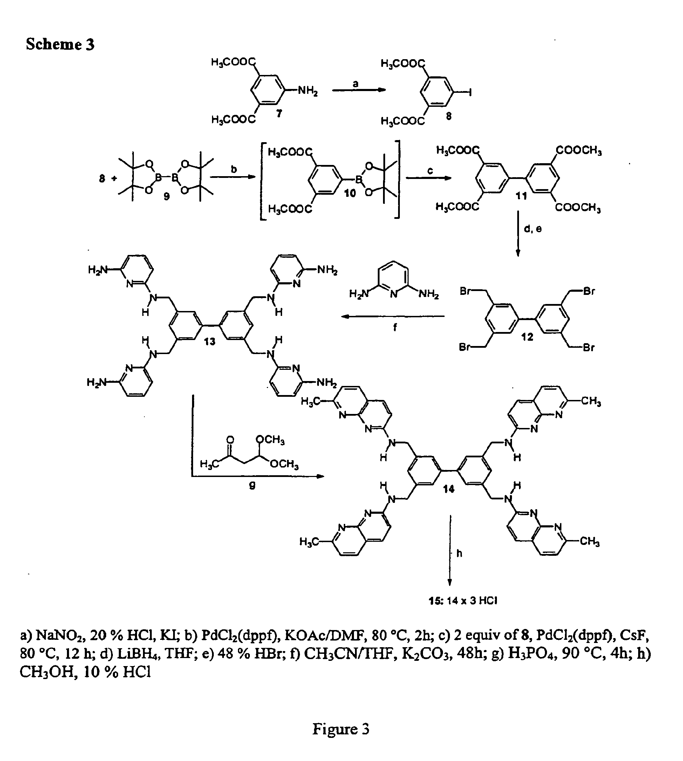

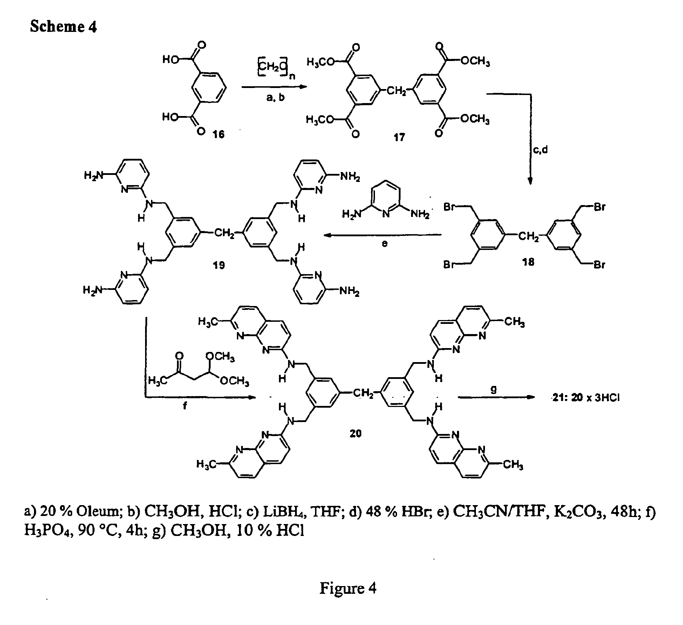

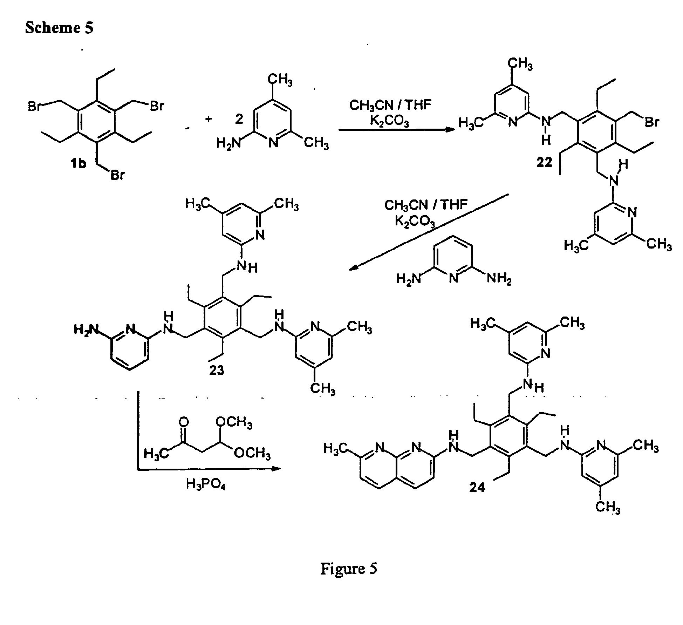

Particularly preferred embodiments are shown in figures 1 to 5.

As used herein, the following definitions will apply unless otherwise indicated:
"Therapeutically effective amount" means a quantity of the compound which is effective in treating the named disorder or condition.

The term "pharmaceutical composition" means a composition suitable for pharmaceutical use in a subject or an individual including an animal or human. A pharmaceutical composition generally comprises an effective amount of an active agent and a carrier, including e.g. a pharmaceutically acceptable carrier.

A "prophylactic treatment" is a treatment administered to a subject or an individual who does not display signs or symptoms of a diseases, pathology, or medical disorder, or displays only early signs of symptoms of disease, pathology or disorders, such that the treatment is administered for the purpose of diminishing, preventing, or decreasing the risk of developing the disease, pathology or medical disorder.

A "therapeutic treatment" is a treatment administered to a subject or an individual who display symptoms or signs of pathology disease, or disorder in which treatment is administered into the subject for the purpose of diminishing or eliminating those signs or symptoms of pathology, disease or disorder.

As used herein, the term "disease", "disorder", "pathology" and "condition" relates to infectious diseases, cancer, tumors or other disease involving binding or recognition of N-acetylneuraminic acid, other sialic acids, or carbohydrates in general. In particular, the disease, disorders, pathology and conditions include but are not limited to bacterial, fungal and viral infections, such as hepatitis B virus, hepatitis C virus, Human Immunodeficiency Virus, FIV influenza, coronavirus, Flavivirus, RSV as well as several others, such as Sendai, Newcastle disease, and polyoma viruses.

As used herein, the term "individual", "patient" or "subject" which is used herein interchangeably refers to an individual or a subject in need of the therapy or prophylaxis. The term "subject", "patient" or "individual" as used herein includes, but is not limited to an organism, a mammal including e.g. a human, a non-human primate (e.g. baboon, monkey), or non-human mammals.

As used herein, the term "carrier" refers to a diluent, adjuvant, excipient or vehicle.

The term "derivative" refers to compounds derived or obtained from another compound and containing essential elements of the parent substance.

The compounds according to the present invention can also be administered in combination with further active ingredients. That is, the pharmaceutical composition according to the present invention may comprise further active ingredients.

The amount of a compound or formula I necessary to achieve the desired biological effect depends on a number of factors the skilled person is well aware of. These factors include but not limited to the specific compounds chosen, the intended use, the mode of administration and the clinical condition of the patient. The pharmaceutical composition of the invention can be produced by one of the known pharmaceutical methods, which essentially consists of mixing the ingredients with pharmacologically acceptable carriers and/or excipient.

### Formulations and derivatives of administration

The pharmaceutical composition according to the present invention containing the compounds of the general formula I are typically administered in a formulation that includes one or more pharmaceutically acceptable carriers or excipients. Such pharmaceutical compositions may be prepared in a manner known per se in the art to result in a pharmaceutical that is sufficiently storagestable and is suitable for administration to humans or animals.

Pharmaceutical acceptable carriers or excipients means a carrier or excipient that at the dosages and concentrations employed does not cause any untoward effects in the patients to whom it is administered. Such pharmaceutically acceptable carriers and excipients are well known in the art (e.g. see Remington's Pharmaceutical Sciences, 18th Edition, A.R. Genaro Ed. Mack, Publishing Company (1990), Handbook of Pharmaceutical Excipients, 3rd Edition, A. Kibbe, Ed. Pharmaceutical Press. 2000).

As indicated before, the term "carrier" or "excipient" refers to a carrier, diluent, adjuvant, excipient or vehicle with which the active ingredient is administered. Such pharmaceutical carriers can be sterile liquid, such as water and oils including those of petroleum, animal, vegetable or synthetic origin, such as peanut oil, soybean oil, mineral oil, sesame oil and the like. Water is a preferred carrier when the pharmaceutical composition is administered intravenously. Saline solutions and glycerol solutions can also be employed as liquid carriers, particularly for injectable solutions.

Suitable pharmaceutical excipient include starch, gelatine, malt, rice, fluor, chalk silica gel, sodium stereate, glycerol monostereate, talc, sodium chloride, dried skim milk, glycerol, propylene, glycol, water, ethanol and the like. Pharmaceutical excipients of the sugar type are less preferred due to possible interaction with the active ingredient. The composition if desired, can also contain minor amounts of wetting or emulsifying agents, or pH buffering agents. These compositions can take the forms of solutions, suspensions, emulsion, tablets, pills, capsules, powders, sustaines release formulations and the like. The composition can be formulated as a suppository with traditional binders and carriers such as glycerides. Oral formulations can include standard carriers, such as pharmaceutical grades of starch, sodium stereate, cellulose, magnesium, carbonate etc. Again, carriers based on sugars are less preferred due to possible interaction with the active principle.

The compounds according to the present invention may be also be a component of a pharmaceutical composition provided in a formulation suitable for parentaral administration, in particular, in subcutaneous, intravenous, intradermal or intramuscular administration.

As noted before, the pharmaceutical composition may be adapted for intravenous administration to human beings. Typically, compositions for intravenous administrations are solutions in sterile isotonic aqueous buffer. Where necessary, the composition may also include a solubilizing agent and a local anaesthetic, such as lidocaine to ease pain at the side of injection.

In vitro assays may optionally be employed to help identifying optimal dosage ranges, the precise dose to be employed in the formulation will also depend on the root of administration, and the seriousness of the disease or disorder, and should be decided according to the judgement of the practitioner and each patient circumstance. Effective doses may be extrapolated from dose response curves derived from in vitro animal model test systems. Preferably, the pharmaceutical composition is administered directly or in combination with an adjuvant.

The term "administered" means administration of a therapeutically effective dose of the aforementioned pharmaceutical composition comprising the compounds according to the present invention, salts and solvates thereof as defined herein to an individual.

The administration of the pharmaceutical composition can be done in a variety of ways as discussed above, including, but not limited to orally, subcutaneously, intravenously, intra-aterial, intranodal, intramedullary, intrathecal, intraventricular, intranasal, conjunctival, intrabronchial, transdermally, intrarectally, intraperitonally, intramusculary, intrapulmonary, vaginally, rectally, or intraocularly.

In still another embodiment, the present invention relates to the use of a compound according to any one of claims 1 to 12 or compounds of the general formula I including stereoisomers and salts or solvates thereof

A-(-CH2 - B)n (I)

wherein
A is
i) a phenyl derivative of the general formula (II) 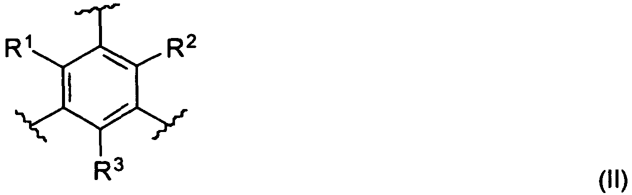 wherein each of R1, R2 and R3 being independently hydrogen or a C1 - C6 alkyl group, a C1 - C6 alkoxy group, a hydroxyl group or a halogen; with the proviso that at least one of R1, R2 or R3 is not hydrogen,
ii) a biphenyl derivative of the general formula (III) 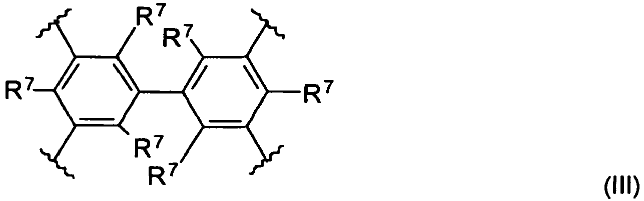 with each R7 being independently a hydrogen or a C1 - C6 alkyl group, a C1 - C6 alkoxy group, a hydroxyl group or a halogen;
iii) a diphenyl alkane derivative of the general formula (IV) 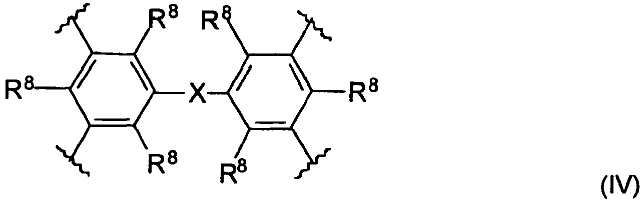 with each R8 is independently a hydrogen or a C1 - C6 alkyl group, a C1 - C6 alkoxy group, a hydroxyl group or a halogen;
X is a C1-C6 alkylen group, in particular, a methylen group;
B is independently an amino-naphthyridine group of the general formula (V), (VI), or (VII) 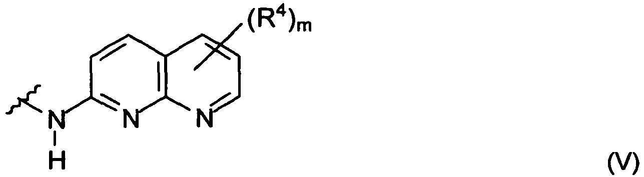 or 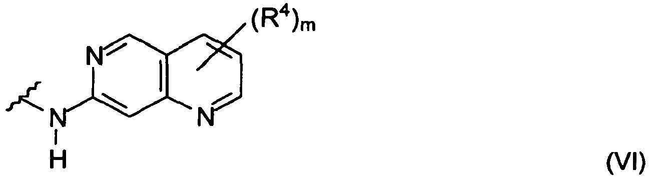 or 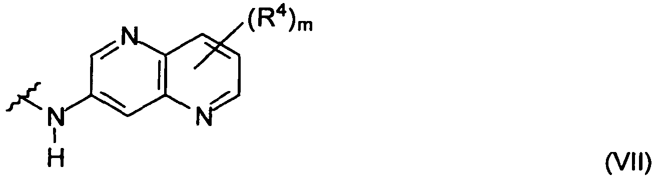 wherein each R4 is independently a hydrogen, a C1-C6 alkyl group, hydroxyl group C1-C6 alkoxy group, halogen or NR10R11 with R10 and R11 being independently hydrogen, or a C1-C6 alkyl group, and m is an integer of 2 to 5,
and n is an integer of 1, 2, or 3 if A is a phenyl derivative of formula (II) or n is 1, 2, 3, or 4 if A is (III) or (IV) and wherein when n is 1 or 2, if A is a phenyl derivative or when n is 1, 2 or 3, if A is a biphenyl derivative or diphenyl-alkane derivative, the spacer moiety is further substituted with any one of the following substituents: -CH2-Y wherein Y is 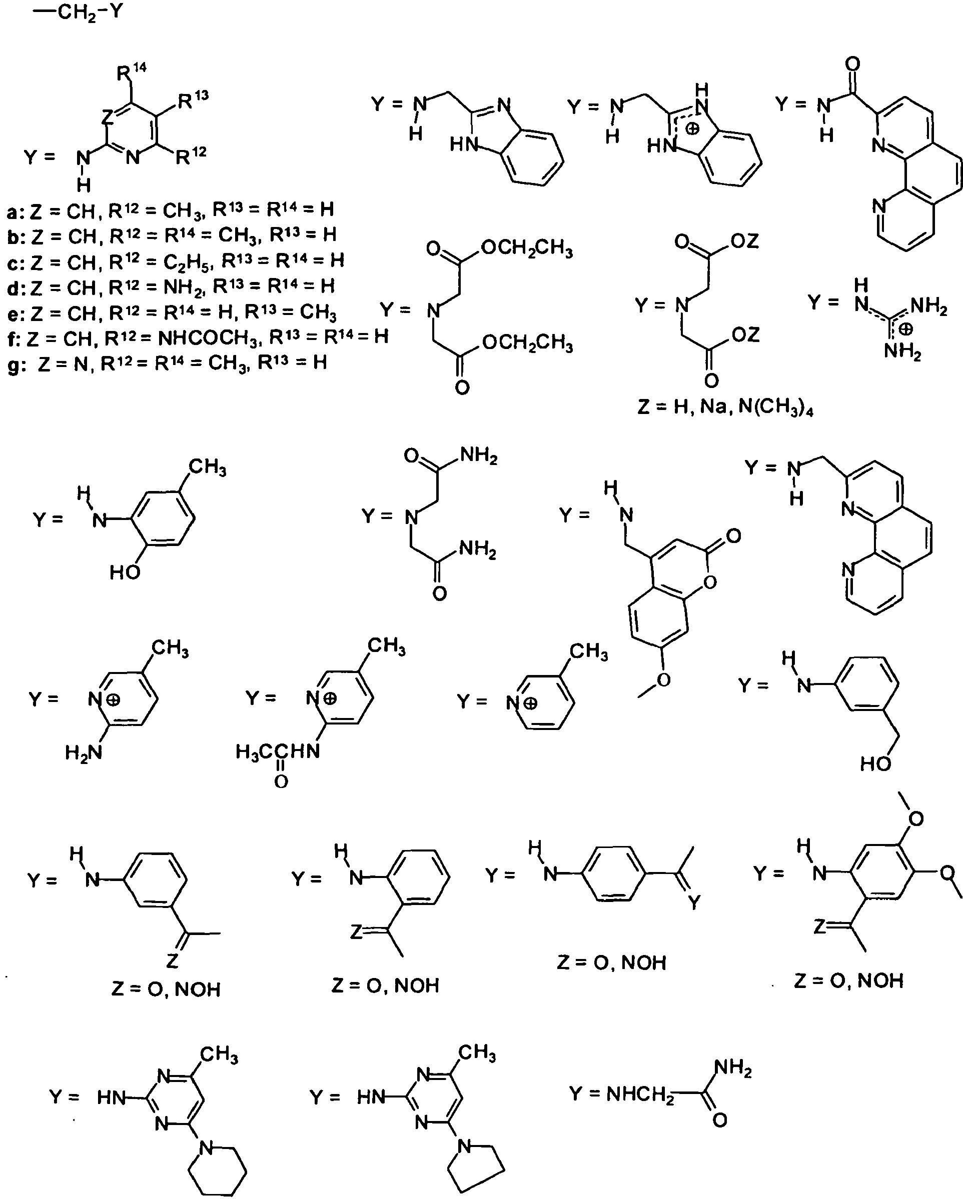 or hydrogen, C1-C6 alkyl group, C1-C6 alkoxy group, hydroxyl group or a halogen, preferably hydrogen, methyl, ethyl, propyl or hydroxyl group, for use in preventing or 5 treating infection or cancer. That is, the present invention relates to the use of said compounds for treating individuals suffering from infectious disease, cancer, tumors or other disease or disorders, comprising the step of administering to said individuals an effective amount of a pharmaceutical composition comprising a compound according to formula I or salts or solvates thereof as the active ingredient, and, optionally, a pharmaceutically acceptable carrier. In particular, the compounds are useful for preventing or treating infectious diseases, like viral, bacterial, parasitic or fungal infections, in particular, hepatitis B, hepatitis C, Human Immunodeficiency Virus, Herpes Virus, influenza or polyoma virus etc.

The term "halogen" refers to a fluorine, chlorine, bromine or iodine atom.

The term "C₁-C₆ alkyl" is used herein as a group or part of the group refers to a linear or branched alkyl group containing from 1 to 6 carbon atoms; examples of such groups include methyl, ethyl, propyl, isopropyl, n-butyl, isobutyl, tertbutyl, pentyl or hexyl.

The solvates may, for example, be hydrates.

The compounds according to the present invention are composed of a spacer unit or spacer moiety as defined above, and, when n is 1 or 2, if A is a phenyl derivative or n is 1, 2 or 3 if A is a biphenyl derivative or diphenyl-alkane derivative, the spacer moiety is optionally further substituted with any one of the following substituents:
CH₂-Y with

### Brief description of the drawings

- Fig.1:: Figure 1, scheme 1, is a scheme showing the synthesis of 1,3,5-Tris[(7-methyl-naphthyridin-2-yl)aminomethyl]-2,4,6-trimethylbenzene (3a) and 1,3,5-Tris[(7-methyl-naphthyridin-2-yl)aminomethyl]-2,4,6-triethylbenzene (3b).
- Fig.2:: Figure 2, scheme 2, shows the synthesis of compounds according to the present invention wherein the naphthyridine residue is substituted with R⁴ and R⁵ are a methyl group.
- Fig.3:: Figure 3, scheme 3, provides a scheme of the synthesis of compounds according to the present invention wherein A is a biphenyl group, n is 4, and the naphthyridine residue is a 2 amino 1,8 naphthyridine with R⁵ is hydrogen and R⁴ is methyl.
- Fig.4:: Figure 4, shows a scheme of the synthesis of compounds according to the present invention. Therein, A is a diphenyl methane derivative, n is 4, and the naphthyridine residue is a 2 amino 1,8 naphthyridine with R⁵ is hydrogen and R⁴ is methyl.
- Fig.5:: Figure 5, scheme 5, shows the synthesis of compounds according to the present invention wherein A is a phenyl derivative wherein R¹, R² and R³ are ethyl, n is 1.

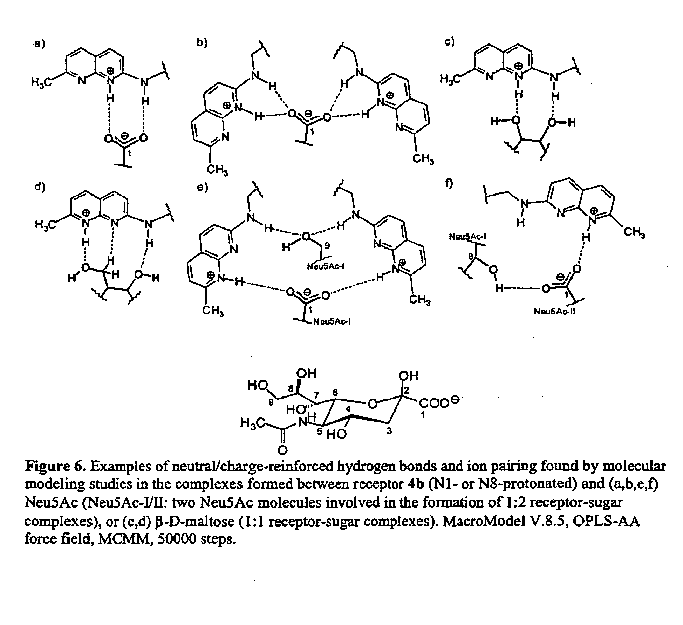

- Fig.6:: Figure 6 provides Examples of neutral/charge-reinforced hydrogen bonds and ion pairing found by molecular modelling studies in complexes formed between receptor 4b and(a, b, e, f) Neu5Ac or (c, d) ß-D-maltose.

The present invention is further described by reference to the following non-limiting figures and examples. The numbers indicated for the title compound refer to the numbering shown in Figs. 1 to 5, respectively.

### Examples

**1,3,5-Tris[(6-amino-pyridin-2-yl)aminomethyl]-2,4,6-trimethylbenzene (2a): A** mixture of 1,3,5-tris(bromomethyl)-2,4,6-trimethyl-benzene (0.72 g, 1.8 mmol), 2,6-diamino-pyridine (1.20 g, 11 mmol), and K₂CO₃ (0.79 g) in CH₃CN (100 mL) was stirred at room temperature for 24 h and then heated under reflux for 2 h. After filtration of the reaction mixture and evaporation of CH₃CN, the obtained powder was suspended in CHCl₃. The suspension was filtrated, the chloroform solution was washed several times with water, dried, and the solvent was removed under reduced pressure. The crude product was crystallized from THF/hexane. Yield 60 %. M.p. 130-132 °C; ¹H NMR (500 MHz, CDCl₃): δ = 2.38 (s, 9H), 4.01 (t, 3H, *J* = 4.4 Hz), 4.15 (s, 6H), 4.35 (d, 6H, *J* = 4.4 Hz), 5.81-5.84 (m, 6H), 7.24 (d, 3H). ¹³C NMR (125 MHz, CDCl₃): δ = 15.83, 41.65, 95.70, 96.81, 133.78, 136.84, 139.25, 157.68, 157.99. HR-MS calcd for C₂₇H₃₃N₉: 483.2859; found: 483.2867.

**1,3,5-Tris[(6-amino-pyridin-2-yl)aminomethyl]-2,4,6-triethylbenzene (2b): A** mixture of 1,3,5-tris(bromomethyl)-2,4,6-triethyl-benzene (0.79 g, 1.8 mmol), 2,6-diamino-pyridine (1.20 g, 11 mmol), and K₂CO₃ (0.79 g) in CH₃CN (100 mL) was stirred at room temperature for 24 h and then heated under reflux for 2 h. After filtration of the reaction mixture and evaporation of CH₃CN, the obtained powder was suspended in CHCl₃. The suspension was filtrated, the chloroform solution was washed several times with water, dried, and the solvent was removed under reduced pressure. The crude product was crystallized from THF/hexane. Yield 40%. M.p. 164-165 °C; ¹H NMR (500 MHz, CDCl₃): δ = 1.22 (t, 9H, *J* = 7.5 Hz), 2.75 (q, 6H, *J* = 7.5 Hz), 4.05 (t, 3H, *J* = 4.2 Hz), 4.20 (s, 6H), 4.35 (d, 6H, *J* = 4.2 Hz), 5.83-5.86 (m, 6H), 7.24 (m, 3H); ¹³C NMR (125 MHz, CDCl₃): δ = 16.9, 23.0, 40.6, 95.9, 96.9, 133.3, 139.4, 143.8, 157.8, 157.9; HR-MS calcd for C₃₀H₃₉N₉: 525.3328; found: 525.3322. R_{f}= 0.21 (CHCl₃/CH₃OH 7:1 *vlv).*

**1,3,5-Tris[(7-methyl-naphthyridin-2-yl)aminomethyl]-2,4,6-trimethylbenzene** (3a): 1,3,5-Tris[(6-amino-pyridin-2-yl)aminomethyl]-2,4,6-trimethylbenzene (2a) (2.0 g, 4.1 mmol), 4,4-dimethoxy-2-butanone (12.3 mmol), and H₃PO₄ (50 mL) were held at 90° for 4 h and then stirred at room temperature for 2 h. The reaction mixture was poured into water (200 mL), neutralized, and extracted several times with chloroform. The collected organic layers were dried over MgSO₄ and the solvent was removed under reduced pressure. The crude product was purified several times by column chromatography (silica gel, CHCl₃/ CH₃OH, 7:1 to 2:1 v/v). Yield 30 %. M.p. 207-208 °C. ¹H-NMR (400 MHz, CDCl₃): δ = 2.34 (s, 9H), 2.61 (s, 9H), 4.71 (d, 6H, 3H, *J* = 3.0 Hz), 5.12 (t, 3H, *J* = 3.0 Hz), 6.60 (d, 3H, *J* = 8.8 Hz), 6.98 (d, 3H, *J* = 8.0 Hz), 7.65 (d, 3H, *J* = 8.8 Hz), 7.74 (d, 3H, *J* = 8.0 Hz). ¹³C NMR (100 MHz, CDCl₃): δ = 15.9, 25.0, 41.1, 112.4, 114.9, 115.9, 133.3, 136.0, 136.5, 137.2, 156.5, 158.7, 161.3. HR-MS calcd for C₃₉H₃₈N₉: 632.3250; found: 632.3256. R_{f} = 0.80 (CHCl₃/CH₃OH 2:1 *vlv).*

**1,3,5-Tris[(7-methyl-naphthyridin-2-yl)aminomethyl]-2,4,6-triethylbenzene (3b):** 1,3,5-Tris[(6-amino-pyridin-2-yl)aminomethyl]-2,4,6-triethylbenzene (2b) (4.7 g, 8.9 mmol), 4,4-dimethoxy-2-butanone (26.3 mmol), and H₃PO₄ (50 mL) were held at 90° for 4 h and then stirred at room temperature for 2 h. The reaction mixture was poured into water (300 mL), neutralized, and extracted several times with chloroform. The collected organic layers were dried over MgSO₄ and the solvent was removed under reduced pressure. The crude product was purified several times by column chromatography (silica gel, CHCl₃/ CH₃OH, 7:1 to 2:1 v/v). Yield 23%. M.p. 207-208 °C. ¹H-NMR (400 MHz, CDCl₃): δ = 1.23 (t, 9H, *J* = 7.5 Hz), 2.69 (s, 9H), 2.82 (q, 6H, *J* = 7.5 Hz), 4.74 (br s, 3H), 4.82 (d, 3H, *J* = 3.0 Hz), 6.58 (d, 3H, *J* = 8.6 Hz), 7.04 (d, 3H, *J* = 8.1 Hz), 7.72 (d, 3H, *J* = 8.6 Hz), 7.79 (d, 3H, *J* = 8.1 Hz). ¹³C NMR (100 MHz, CDCl₃): δ =16.9, 23.1, 25.3, 40.6, 112.1, 115.2, 118.3, 132.9, 136.2, 136.9, 144.5, 156.7, 158.4, 161.3. HR-MS calcd for C₄₂H₄₄N₉: 674.3719; found: 674.3714. R_{f} = 0.89 (CHCl₃/CH₃OH 2:1 *vlv).*

**Trihydrochloride 4b:** Compound **3b** (0.1 g, 0.148 mmol) was dissolved in MeOH (3 mL), and HCl (1 mL, 10 %) was added. The resulting solution was stirred at room temperature for 30 min and evaporated in *vacuo.* This procedure was repeated twice to obtain the trihydrochloride **3b.** M.p. 237-238 °C. ¹H NMR (400 MHz, DMSO-d₆): δ = 1.16 (t, 9H, *J* = 9.9 Hz), 2.78-2.82 (s + q, 9H + 6H), 4.80 (d, 3H, *J* = 3.5 Hz), 7.33 (d, 3H, *J* = 12.1 Hz), 7.48 (d, 3H, *J* = 10.6 Hz), 8.15 (d, 3H, *J* = 12.1 Hz), 8.61 (d, 3H, *J* = 10.6 Hz), 9.08 (br s, 3H), 15.29 (br s, 3H). ¹³C NMR (100 MHz, DMSO-d₆): δ = 15.9, 19.5, 22.8, 39.5, 116.1, 116.8, 117.8, 130.7, 136.2, 143.7, 149.5, 154.8. 159.5. ESI-MS: m/z = 676 [M-2H⁺]⁺, 338.6 [M-H⁺]²⁺, 226.1 [M]³⁺ (C₄₂H₄₈N₉³⁺).

**Compound 22.** To a mixture of 1,3,5-tris(bromomethyl)-2,4,6-trimethyl-benzene (**1b**) (3.00 g, 6.80 mmol) and K₂CO₃ (1.88 g, 13.60 mmol) in CH₃CN/THF (1:1 v/v; 40 mL) was added dropwise a CH₃CN (10 mL) solution of 2-amino-4,6-dimethyl-pyridine (1.66 g, 13.60 mmol).The mixture was stirred at room temperature for 72 h. After filtration and evaporation of solvents, the crude product was purified by column chromatography (ethyl acetate/toluene, 1:3 v/v). Yield 30 %. M.p. 77-78 °C. ¹H-NMR (400 MHz, CDCl₃) δ = 1.22 (t, 3H, *J* = 7.5 Hz), 1.29 (t, 6H, *J* = 7.5 Hz), 2.24 (s, 6H), 2.36 (s, 6 H), 2.73 (q, 2H, *J* = 7.5 Hz), 2.85 (q, 4H, *J* = 7.5 Hz), 4.23 (t, 2H, *J* = 4.2 Hz), 4.37 (d, 4H, *J* = 4.2 Hz), 4.62 (s, 2 H), 6.10 (s, 2 H), 6.35 (s, 2 H). ¹³C-NMR (100 MHz, CDCl₃) δ = 16.4, 16.7, 21.1, 22.8, 23.0, 24.1, 29.6, 40.5, 103.6, 113.9, 131.9, 133.4, 143.8, 144.9, 148.9, 156.5, 158.0. HR-MS calcd for C₂₉H₃₉BrN₄ 5232.2353; found: 522.2360. R_{f} = 0.31 (ethyl acetate/toluene, 1:3 v/v).

**Compound 23.** To a mixture of 2,6-diaminopyridine (0.50 g, 4.58 mmol) and K₂CO₃ (0.21 g, 1.53 mmol) in CH₃CN (10 mL) was added a CH₃CN/THF (1:1 v/v; 20 mL) solution of compound **22** (0.9 g, 1.53 mmol). The mixture was heated to reflux for 12 h and stirred at room temperature for additional 24 h. After filtration and evaporation of solvents the crude product was taken up in CHCl₃ (20 mL) and washed several times with water. The organic phase was dried over MgSO₄ and the solvent was evaporated. The crude product was purified by column chromatography (toluene /ethyl acetate 1:3 (v:v) containing 2 % triethylamine). Yield 62 %. M.p. 78-79 °C. ¹H-NMR (400 MHz, CDCl₃) δ = 1.23 (t, ³*J* = 7.5 Hz, 9 H), 2.23 (s, 6 H), 2.35 (s, 6 H), 2.74 (q, ³*J* = 7.5 Hz, 6 H), 4.01 (br. s, 1 H), 4.13 (br. s, 2 H), 4.18 (br. s, 2 H), 4.35 (d, ³*J* = 4.3 Hz, 2 H), 4.37 (d, ³*J* = 4.3 Hz, 4 H), 5.83 (d, ³*J* = 6.0 Hz, 1 H), 5.85 (d, ³*J* = 5.7 Hz, 1 H), 6.11 (s, 2 H), 6.34 (s, 2 H), 7.25 (t, ³*J* = 7.8 Hz, 1 H). ¹³C-NMR (100 MHz, CDCl₃) δ = 16.85, 21.09, 22.88, 24.21, 40.47, 40.63, 95.89, 96.67, 103.46, 113.90, 133.18, 143.53, 143.58, 139.18, 148.72, 156.76, 157.71, 157.85, 158.24. HR-MS calcd for C₃₄H₄₅N₇ 551.3736; found: 551.3742. R*_{f}* = 0.3 (chloroform/methanol, 7:1 v/v)

**Compound 24.** Compound **23** (100 mg, 0.18 mmol), 4,4-dimethoxybutanone (0.016 mL, 0.36 mmol) and H₃PO₄ (5 mL) were held at 90°C for 2.5 h and then stirred at room temperature for 2 h. The reaction mixture was poured into water (10 mL), neutralized nad extracted several times with chloroform. The collected organic layers were dried over MgSO₄ and the solvent was removed under reduced pressure. The crude product was purified several times by column chromatography (chloroform/methanole 20:1 v/v). Yield 16.5 %. ¹H-NMR (400 MHz, CDCl₃) = 1.23 (m, 9H), 2.23 (s, 6H), 2.34 (s, 6H), 2.72 (s, 3H), 2.80 (m, 6H), 4.26 (s, 2H), 4.37 (d, J = 4.1 Hz, 4H), 4.62 (s, 1H), 4.79 (d, J = 3.8 Hz, 2H), 6.11 (s, 2H), 6.35 (s, 2H), 6.60 (d, J = 8.7 Hz, 1 H) 7.05 (d, J = 8.0 Hz, 1 H), 7.72 (d, J = 8.7 Hz, 1 H), 7.80 (d, J = 8.0 Hz, 1 H).

## Claims

1. Compounds of the general formula (I) including stereoisomers and salts or solvates thereof
A-(-CH2 - B)n (I)
wherein
A is a spacer moiety selected from
i) a phenyl derivative of the general formula (II) 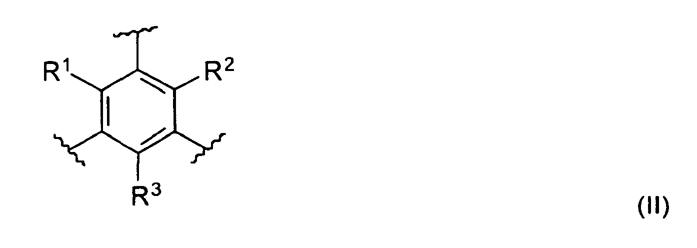 wherein each of R1, R2 and R3 being independently a C1 - C6 alkyl group, a C1 - C6 alkoxy group, a hydroxyl group or a halogen;
ii) a biphenyl derivative of the general formula (III) 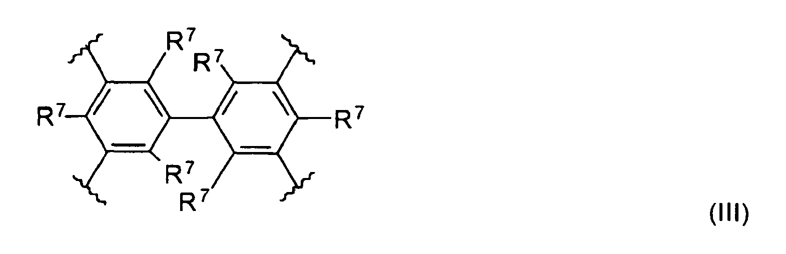 with each R7 being independently a hydrogen or a C1 - C6 alkyl group, a C1 - C6 alkoxy group, a hydroxyl group or a halogen;
iii) a diphenyl alkane derivative of the general formula (IV) 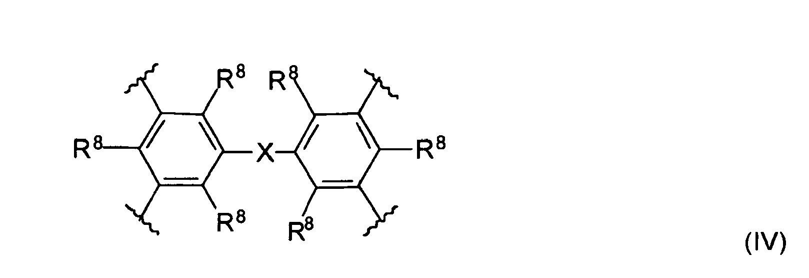 with each R8 is independently a hydrogen or a C1 - C6 alkyl group, a C1 - C6 alkoxy group, a hydroxyl group or a halogen;
X is a C1-C6 alkylen group, in particular, a methylen group;
B is independently an amino-naphthyridine group of the general formula (V), (VI), or (VII) 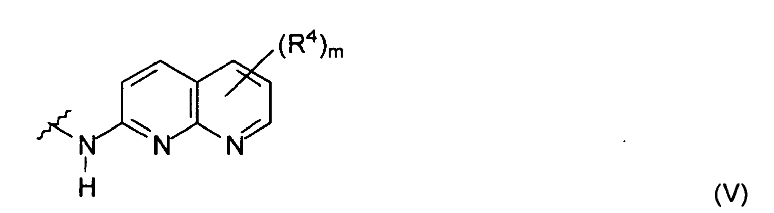 or 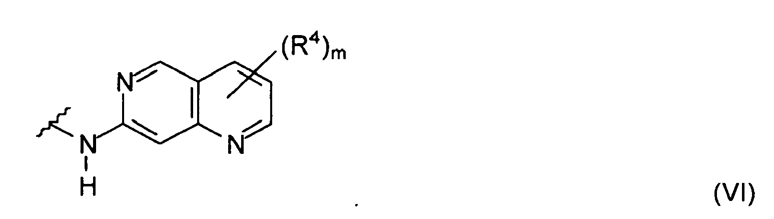 or 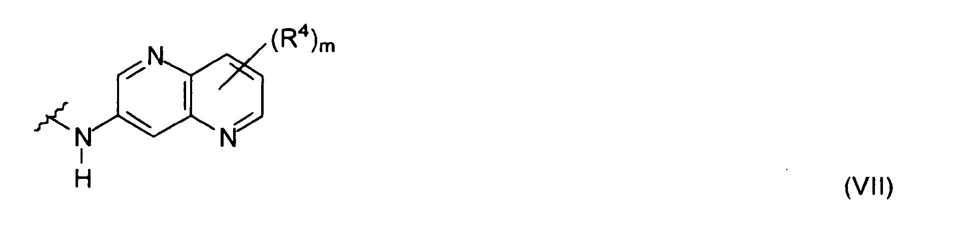 wherein each R4 is independently a hydrogen, a C1-C6 alkyl group,
hydroxyl group C1-C6 alkoxy group, halogen or NR10R11 with R10 and R11 being independently hydrogen, or a C1-C6 alkyl group, and m is an integer of 2 to 5,
and n is an integer of 1, 2, or 3 if A is a phenyl derivative of formula (II) or n is 1, 2, 3, or 4 if A is (III) or (IV), and wherein when n is 1 or 2, if A is a phenyl derivative or when n is 1, 2 or 3, if A is a biphenyl derivative or diphenyl-alkane derivative, the spacer moiety is further substituted with any one of the following substituents:
-CH2-Y wherein Y is 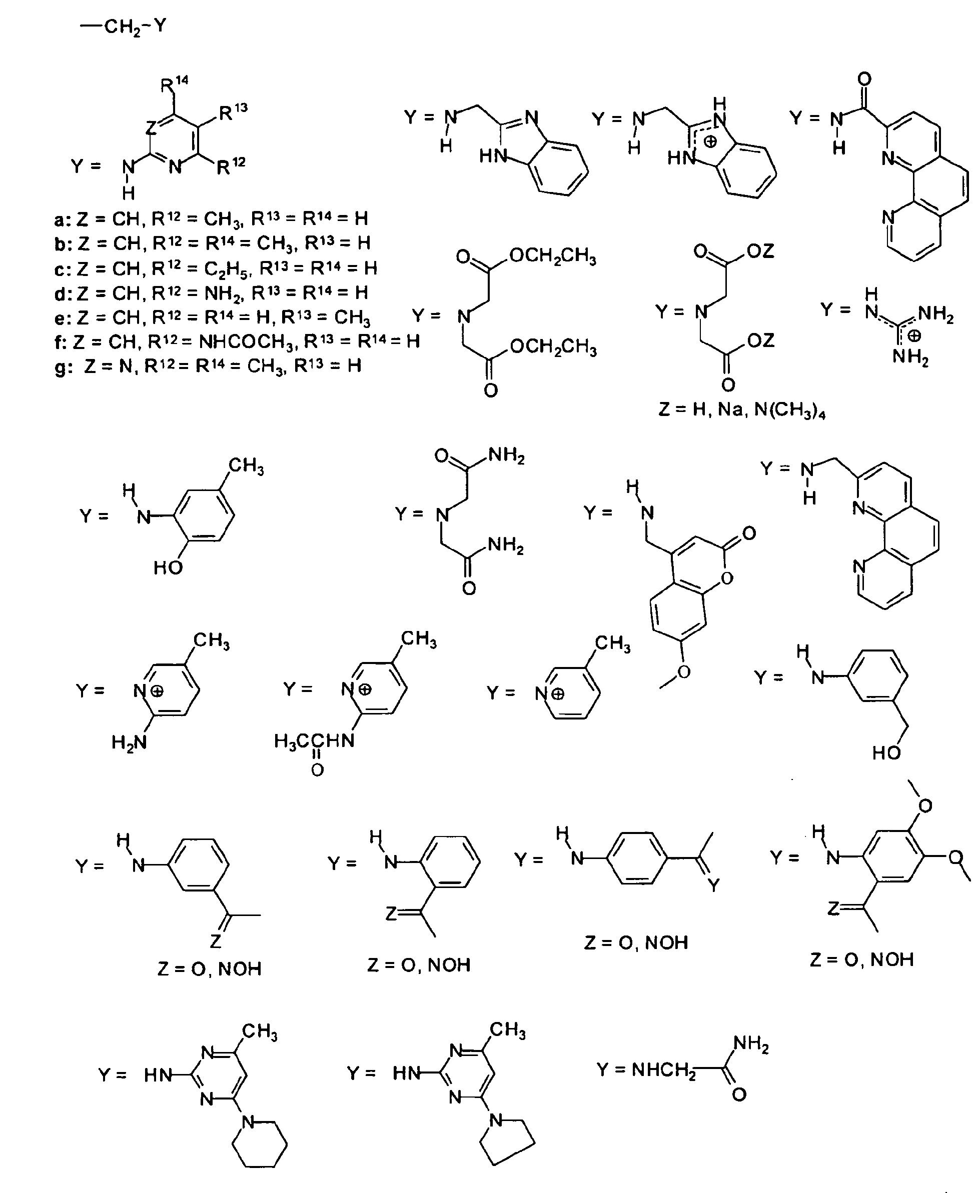
, or hydrogen, C1-C6 alkyl group, C1-C6 alkoxy group, hydroxyl group or a halogen, preferably hydrogen, methyl, ethyl, propyl or hydroxyl group.

2. A compound according to claim 1, wherein
B is an amino-naphthyridine group of the general formula (Va), (VIa), or (VIIa) 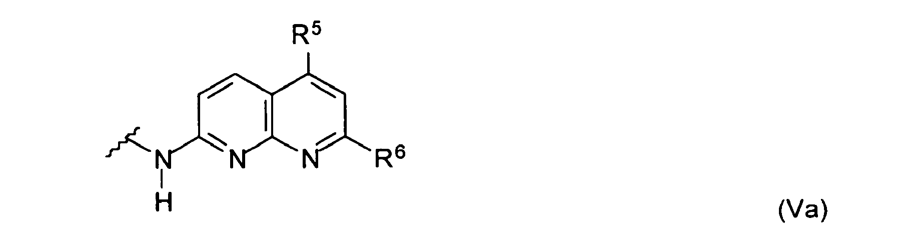 or 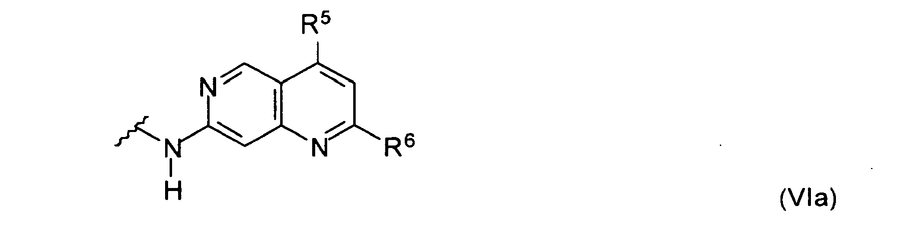 or 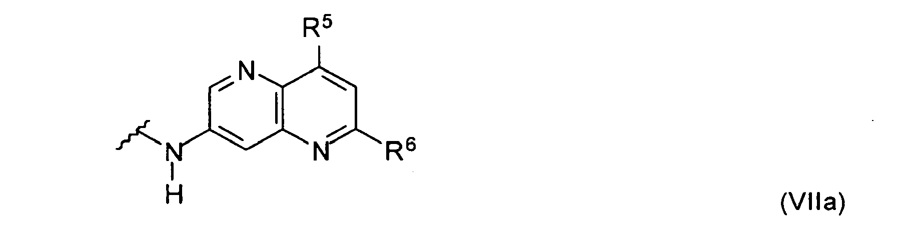 wherein R5 or R6 being independently hydrogen, a C1-C6 alkyl group, hydroxyl group, C1-C6 alkoxy group, halogen or NR10R11 with R10 and R11 being independently hydrogen, or a C1-C6 alkyl group.

3. A compound according to claim 1 or 2, wherein A is a phenyl derivative of the general formula II and each R1, R2 and R3 are independently an alkyl group selected from the group of methyl, ethyl, propyl or butyl.

4. A compound according to claim 3, wherein each R1, R2 and R3 are a methyl or ethyl group.

5. A compound according to claim 1 or 2, wherein A is the biphenyl derivative of the general formula III and each R7 are independently hydrogen, methyl, ethyl, propyl, methoxy or ethoxy groups.

6. A compound according to claim 1 or 2, wherein A is a diphenyl alkane derivative of the general formula IV and each R8 is independently hydrogen, methyl, ethyl, propyl, hydroxyl, methoxy or ethoxy groups, in particular, methyl groups.

7. A compound according to any one of claims 1,3 to 6, wherein each R4 is independently hydrogen, or a methyl, ethyl, propyl, isopropyl or butyl group.

8. A compound according to any one of claims 1 to 6, wherein R5 or R6 being independently hydrogen or methyl, ethyl, propyl, isopropyl or butyl group.

9. A compound according to claim 8, wherein R6 is methyl and R5 is hydrogen or methyl.

10. A compound according to any one of the preceding claims, wherein A is a phenyl derivative of the general formula II and n is 3.

11. A compound according to claim 1 wherein A is a biphenyl derivative of the general formula III or a diphenyl alkane derivative of the general formula IV wherein n is 4.

12. A compound according to any one of the preceding claims which is a halogen salt.

13. Pharmaceutical composition comprising a compound according to any one of claims 1 to 12 and, optionally, a pharmaceutically acceptable carrier or excipient.

14. A compound according to any one of claims 1 to 12 or compounds of the general formula I including stereoisomers and salts or solvates thereof
A-(-CH2 - B)n (I)
wherein
A is
i) a phenyl derivative of the general formula (II) 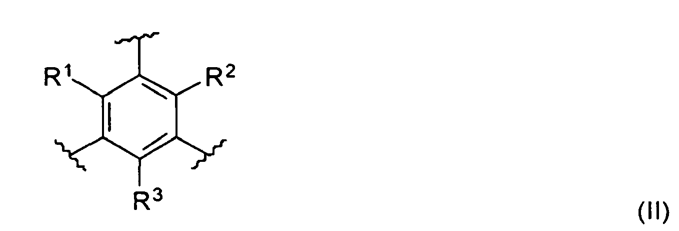 wherein each of R1, R2 and R3 being independently hydrogen or a C1 - C6 alkyl group, a C1 - C6 alkoxy group, a hydroxyl group or a halogen; with the proviso that at least one of R1, R2 or R3 is not hydrogen,
ii) a biphenyl derivative of the general formula (III) 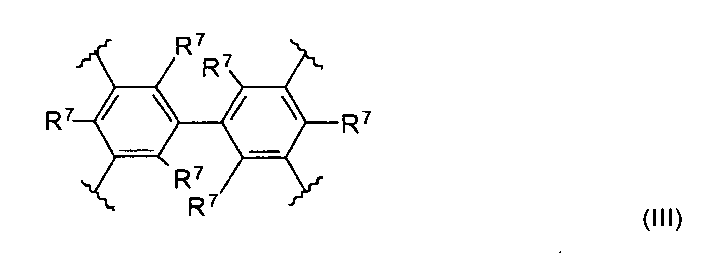 with each R7 being independently a hydrogen or a C1 - C6 alkyl group, a C1 - C6 alkoxy group, a hydroxyl group or a halogen;
iii) a diphenyl alkane derivative of the general formula (IV) 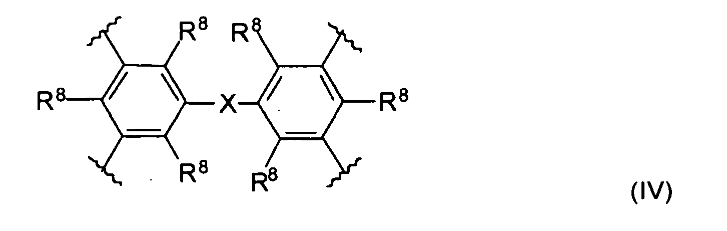 with each R8 is independently a hydrogen or a C1 - C6 alkyl group, a C1 - C6 alkoxy group, a hydroxyl group or a halogen;
X is a C1-C6 alkylen group, in particular, a methylen group;
B is independently an amino-naphthyridine group of the general formula (V), (VI), or (VII) 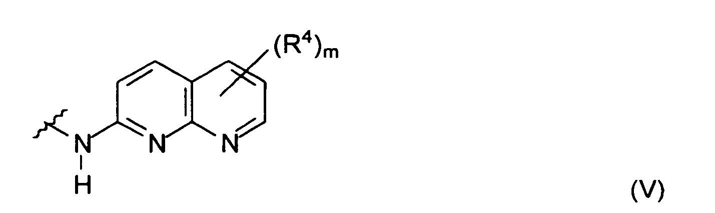 or 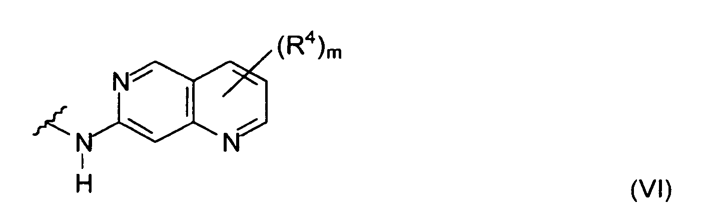 or 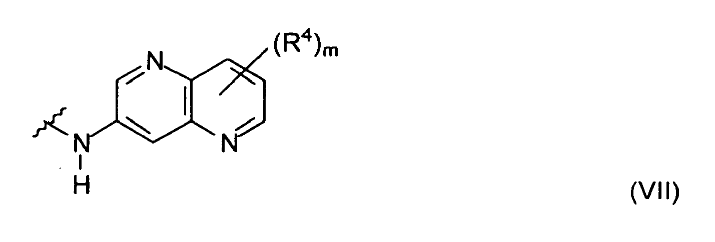 wherein each R4 is independently a hydrogen, a C1-C6 alkyl group, hydroxyl group C1-C6 alkoxy group, halogen or NR10R11 with R10 and
R11 being independently hydrogen, or a C1-C6 alkyl group, and m is an integer of 2 to 5,
and n is an integer of 1, 2, or 3 if A is a phenyl derivative of formula (II) or n is 1, 2, 3, or 4 if A is (III) or (IV) and wherein when n is 1 or 2, if A is a phenyl derivative or when n is 1, 2 or 3, if A is a biphenyl derivative or diphenyl-alkane derivative, the spacer moiety is further substituted with any one of the following substituents:
- CH2-Y wherein Y is 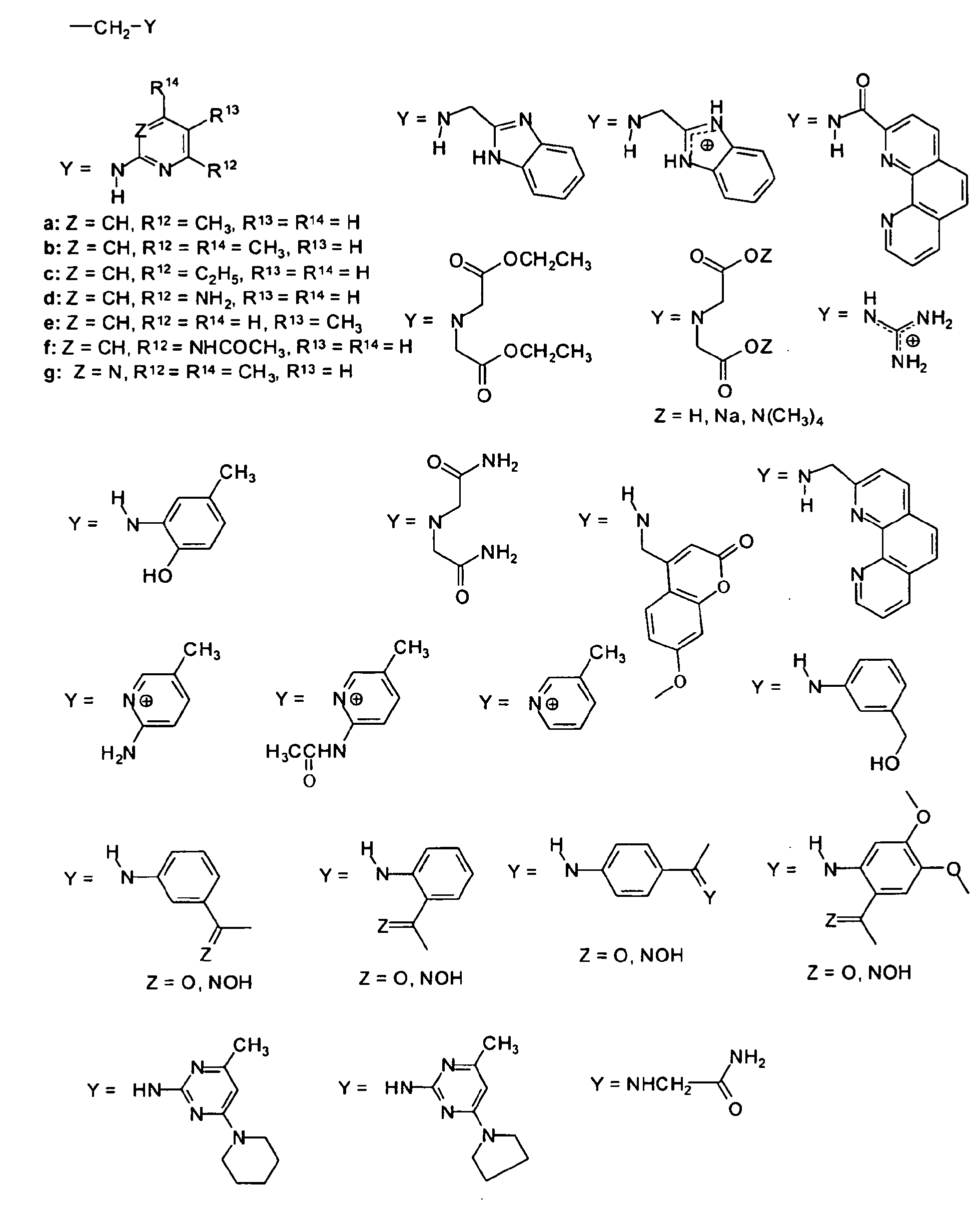
, or hydrogen, C1-C6 alkyl group, C1-C6 alkoxy group, hydroxyl group or a halogen, preferably hydrogen, methyl, ethyl, propyl or hydroxyl group, for use in preventing or treating infection or cancer.

## Patentansprüche

1. Verbindung der allgemeinen Formel (I) einschließlich Stereoisomere und Salze oder Solvate hiervon:
A-(-CH₂ - B)n (I)
wobei
A eine Abstandseinheit ist ausgewählt aus
i) ein Phenylderivat der allgemeinen Formel (II) 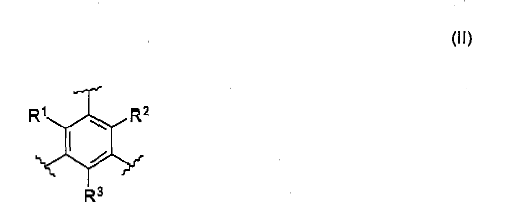 wobei jedes von R1, R2 und R3 unabhängig voneinander eine C1 - C6 Alkylgruppe, eine C1 - C6 Alkoxygruppe, eine Hydroxylgruppe oder ein Halogen ist;
ii) ein Biphenylderivat der allgemeinen Formel (III) 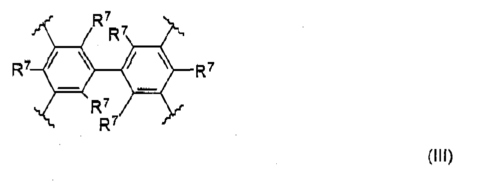 wobei jedes von R7 unabhängig voneinander einen Wasserstoff oder eine C1 - C6 Alkylgruppe, C1 - C6 Alkoxygruppe, eine Hydroxylgruppe oder ein Halogen ist;
iii) ein Diphenylalkanderviat der allgemeinen Formel (IV) 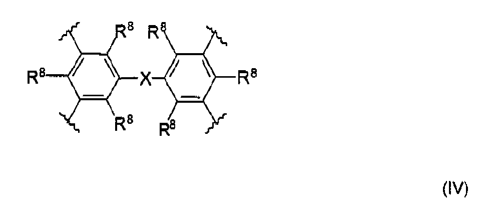 wobei jedes R8 unabhängig voneinander einen Wasserstoff oder eine C1 - C6 Alkylgruppe, eine C1 - C6 Alkoxygruppe, eine Hydroxylgruppe oder ein Halogen ist;
X ist eine C1 - C6 Alkylengruppe, insbesondere eine Methylengruppe;
B ist unabhängig voneinander eine Amino-Naphtyridingruppe der allgemeinen Formel (V), (VI) oder (VII) 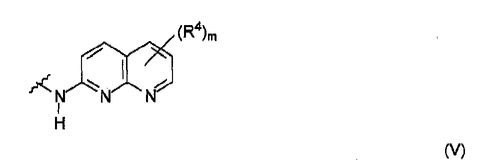 oder 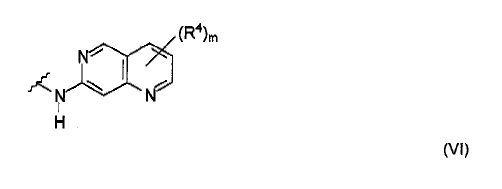 oder 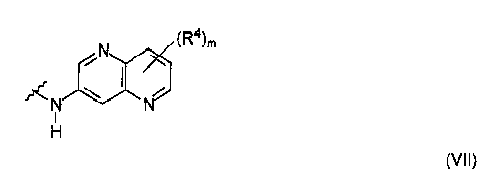 wobei jedes der R4 unabhängig voneinander ein Wasserstoff, eine C1 - C6 Alkylgruppe, eine Hydroxylgruppe, eine C1 - C6 Alkoxygruppe, Halogen oder NR¹⁰R¹¹ mit R¹⁰ und R¹¹ unabhängig voneinander Wasserstoff oder eine C1 - C6 Alkylgruppe, ist, und m ist eine ganze Zahl von 2 bis 5,
und n ist eine ganze Zahl von 1, 2 oder 3, wenn A ein Phenylderivat der Formel (II) ist oder n Ist 1, 2, 3 oder 4 wenn A (III) oder (VI) ist, und wobei wenn n 1 oder 2 ist, wenn A ein Phenylderivat ist oder wenn n 1, 2 oder 3 ist, wenn A ein Diphenylalkalderviat oder Diphenylalkanderivat ist, die Abstandseinheit weiter substituiert ist mit einem der folgenden Substituenten.
-CH₂-Y, wobei Y ist 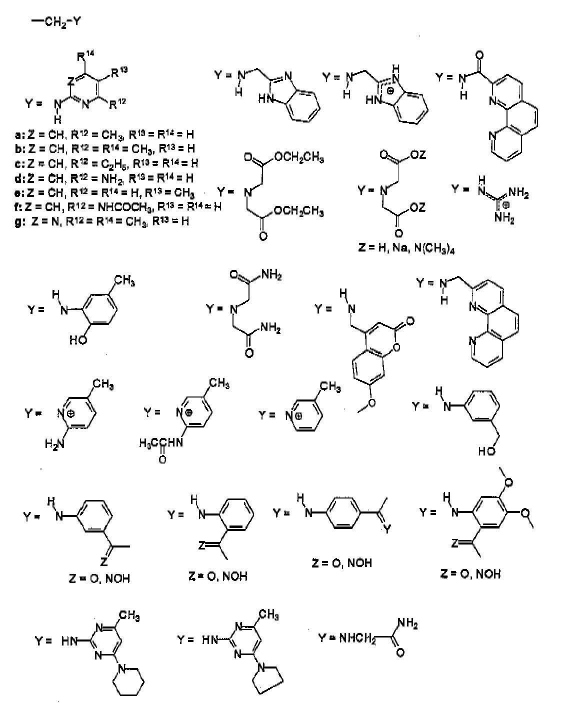 ,oder Wasserstoff, C1 - C6 Alkylgruppe, C1 - C6 Alkoxygruppe, Hydroxylgruppe oder ein Halogen, bevorzugt Wasserstoff, Methyl, Ethyl, Propyl oder Hydroxylgruppe.

2. Verbindung nach Anspruch 1, wobei B eine Amino-Naphtyridingruppe der allgemeinen Formel (Va), (Via) oder (Vlla) ist 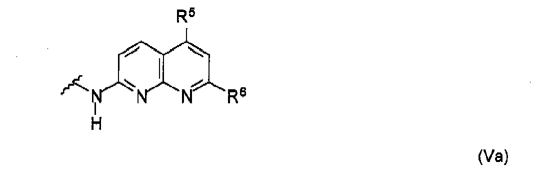 oder 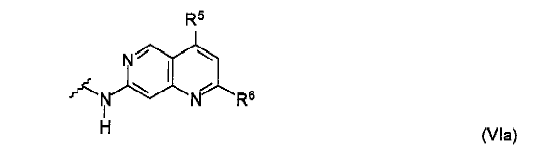 oder 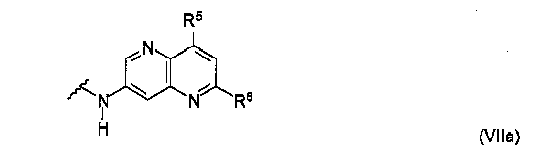 wobei R5 oder R6 unabhängig voneinander Wasserstoff, eine C1 - C6 Alkylgruppe, eine Hydroxylgruppe, eine C1 - C6 Alkoxygruppe, ein Halogen oder NR¹⁰R¹¹, mit R¹⁰ oder R¹¹ unabhängig voneinander Wasserstoff oder eine C1 - C6 Alkylgruppe ist.

3. Verbindung nach Anspruch 1 oder 2, wobei A ein Phenylderivat der allgemeinen Formel (II) ist und jedes von R1, R2 und R3 unabhängig voneinander eine Alkylgruppe, ausgewählt aus einer Gruppe von Methyl, Ethyl, Propyl oder Butyl.

4. Verbindung nach Anspruch 3, wobei jedes von R1, R2 und R3 eine Methyl- oder Ethylgruppe ist.

5. Verbindung nach Anspruch 1 oder 2, wobei A das Biphenylderivat der allgemeinen Formel (III) Ist und jedes von R7 ist unabhängig voneinander Wasserstoff, Methyl-, Ethyl-, Propyl-, Methoxy- oder Ethoxygruppen.

6. Verbindung nach Anspruch 1 oder 2, wobei A ein Diphenylalkanderivat der allgemeinen Formel (IV) ist und jedes von R8 ist unabhängig voneinander Wasserstoff, Methyl-, Ethyl-, Propyl-, Hydroxyl-, Methoxy- oder Ethoxygruppen, insbesondere Methylgruppen.

7. Verbindung nach einem der Ansprüche 1, 3 bis 6, wobei jedes von R4 unabhängig voneinander Wasserstoff, eine Methyl-, Ethyl-, Propyl-, Isopropyl- oder Butylgruppe ist.

8. Verbindung gemäß einem der Ansprüche 1 bis 6, wobei R5 oder R6 unabhängig voneinander Wasserstoff oder Methyl-, Ethyl-, Propyl-, Isopropyl- oder Butylgruppe ist.

9. Verbindung nach Anspruch 8, wobei R6 Methyl ist und R5 ist Wasserstoff oder Methyl.

10. Verbindung nach einem der vorherigen Ansprüche, wobei A ein Phenylderivat der allgemeinen Formel (II) ist und n ist 3.

11. Verbindung nach Anspruch 1, wobei A ein Biphenylderivat der allgemeinen Formel (III) ist oder ein Diphenylalkalderviat der allgemeinen Formel (IV) und wobei n 4 ist.

12. Verbindung nach einem der vorhergehenden Ansprüche, dass es ein Halogensalz ist.

13. Pharmazeutische Zusammensetzung umfassend eine Verbindung nach einem der Ansprüche 1 bis 12 und ggf. ein pharmazeutisch annehmbaren Träger oder Excipienten.

14. Verbindung nach einem der Ansprüche 1 bis 12 oder Verbindung der allgemeinen Formel (I) einschließlich Stereoisomere und Salz oder Solvate hiervon.
A-(-CH₂ - B)n (I)
wobei
A ist
i) ein Phenylderivat der allgemeinen Formel (II) 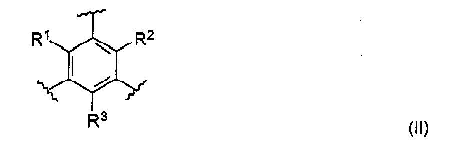 wobei jedes von R1, R2 und R3 unabhängig voneinander Wasserstoff oder eine C1 - C6 Alkylgruppe, eine C1 - C6 Alkoxygruppe, eine Hydroxylgruppe und ein Halogen ist, unter der Bedingung, dass mindestens eins von R1, R2 oder R3 kein Wasserstoff ist,
ii) ein Biphenylderivat der allgemeinen Formel III 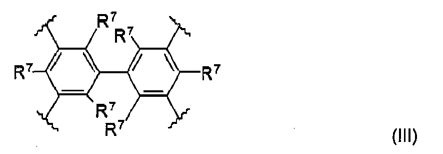 wobei jedes von R7 unabhängig voneinander ein Wasserstoff oder eine C1 - C6 Alkylgruppe, eine C1 - C6 Alkoxygruppe, eine Hydroxylgruppe oder ein Halogen ist;
iii) ein Diphenylalkanderviat der allgemeinen Formel (IV) 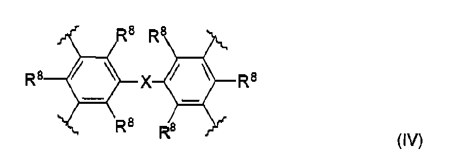 wobei jedes R8 unabhängig voneinander einen Wasserstoff oder eine C1 - C6 Alkylgruppe, eine C1 - C6 Alkoxygruppe, eine Hydroxylgruppe oder ein Halogen ist;
X ist eine C1 - C6 Alkylengruppe, insbesondere eine Methylengruppe;
B ist unabhängig voneinander eine Amino-Naphtyridingruppe der allgemeinen Formeln (V), (VI) oder (VIII) 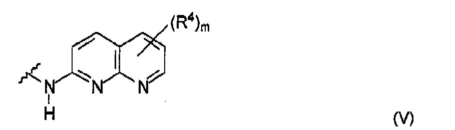 ou 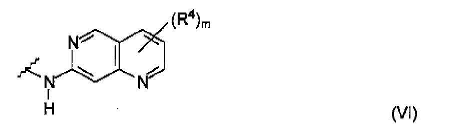 ou 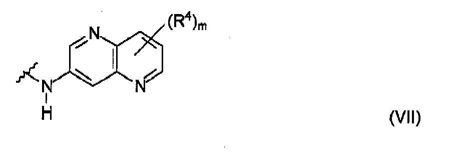 wobei jedes R4 unabhängig voneinander einen Wasserstoff, eine C1 - C6 Alkylgruppe, eine Hydroxylgruppe, eine C1 - C6 Alkoxygruppe, Halogen oder NR¹⁰R¹¹ mit R¹⁰ und R¹¹ unabhängig voneinander einen Wasserstoff, eine C1 - C6 Alkylgruppe ist und m ist eine ganze Zahl von 2 bis 5, und n ist eine ganze Zahl von 1, 2 oder 3 wenn A ein Phenylderivat der Formel (II) ist oder n ist 1, 2, 3 oder 4 wenn A (III) oder (IV) ist und wobei wenn n 1 oder 2 Ist, wenn A ein Phenylderivat ist oder wenn n 1, 2 oder 3 ist, wenn A ein Biphenylderivat oder ein Diphenylalkalderviat Ist, die Abstandseinheit weiter substituiert ist mit mindestens einem der folgenden Substituenten:
CH₂-Y wobei Y ist 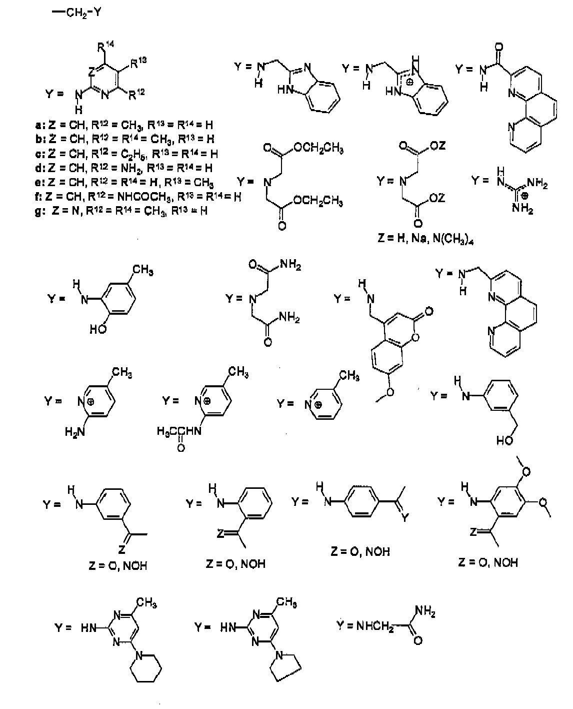 , oder Wasserstoff, C1 - C6 Alkylgruppe, C1 - C6 Alkoxygruppe, Hydroxylgruppe oder ein Halogen, bevorzugt Wasserstoff, Methyl, Ethyl, Propyl oder Hydroxylgruppe, zur Verwendung in der Vorbeugung oder Behandlung von Infektionen oder Krebs.

## Revendications

1. Composés de formule générale (I), y compris les stéréoisomères et les sels et solvates de ceux-ci
A- (-CH₂ - B)ₙ (I)
dans laquelle
A est un motif espaceur choisi parmi
i) un dérivé phényle de formule générale (II) 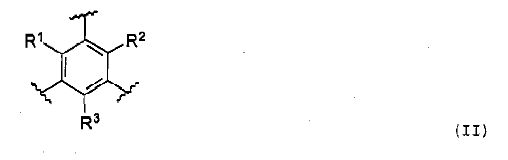 chacun de R¹, R² et R³ étant indépendamment un groupement alkyle en C₁ - C₆, un groupement alcoxy en C₁ - C₆, un groupement hydroxyle ou un halogène ;
ii) un dérivé biphényle de formule générale (III) 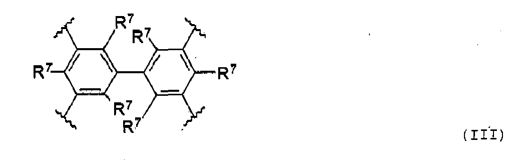 chaque R⁷ étant indépendamment un hydrogène ou un groupement alkyle en C₁ - C₆, un groupement alcoxy en C₁ - C₆, un groupement hydroxyle ou un halogène ;
iii) un dérivé diphénylalcane de formule générale (IV) 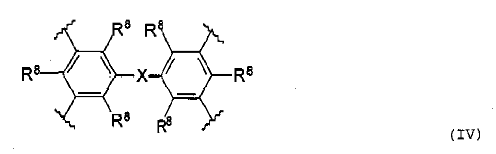 chaque R⁸ étant indépendamment un hydrogène ou un groupement alkyle en C₁ - C₆, un groupement alcoxy en C₁ - C₆, a groupement hydroxyle ou un halogène ; X étant un groupement alkylène en C₁-C₆, en particulier un groupement méthyléne ;
B est indépendamment un groupement amino-naphtyridine de formule générale (V), (VI) ou (VII) 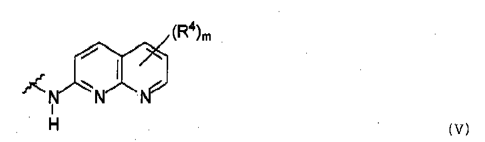 ou 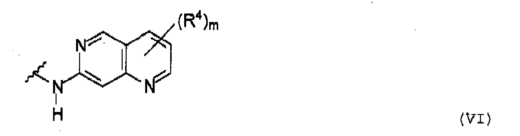 ou 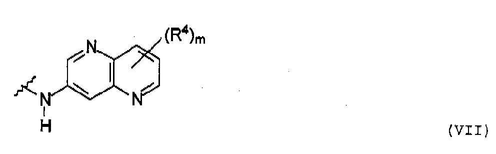 chaque R⁴ étant indépendamment un hydrogène, un groupement alkyle en C₁-C₆, un groupement hydroxyle, un groupement alcoxy en C₁-C₆, un halogène ou NR¹⁰R¹¹, R¹⁰ et R¹¹ étant indépendamment hydrogène, ou un groupement alkyle en C₁-C₆, et m étant un entier de 2 à 5,
et n est un entier de 1, 2 ou 3 si A est un dérivé phényle de formule (II) ou n est 1, 2, 3 ou 4 si A est (III) ou (IV), et dans laquelle lorsque n est 1 ou 2, si A est un dérivé phényle ou lorsque n est 1, 2 ou 3, si A est un dérivé biphényle ou un dérivé diphénylalcane, le motif espaceur est substitué en outre par l'un quelconque des substituants suivants :
- CH₂-Y où Y est 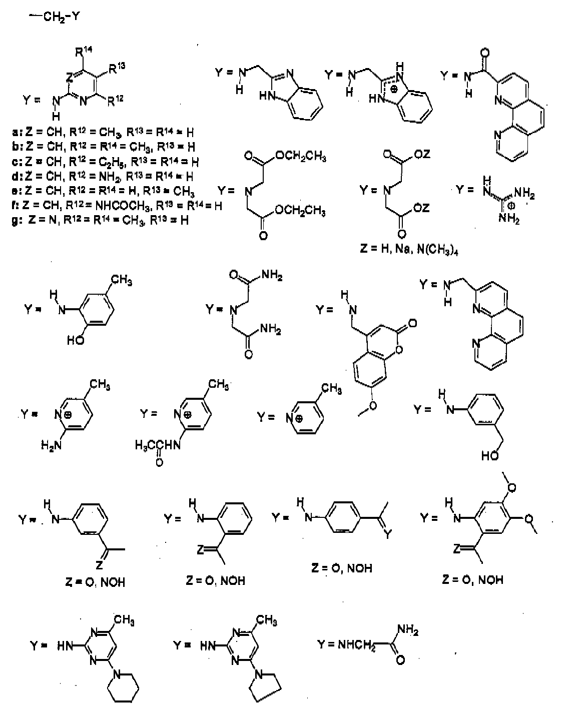
ou hydrogène, un groupement alkyle en C₁-C₆, un groupement alcoxy en C₁-C₆, un groupement hydroxyle ou un halogène, de préférence hydrogène, un groupement méthyle, éthyle, propyle ou hydroxyle.

2. Composé selon la revendication 1, **caractérisé en ce que**
B est un groupement amino-naphtyridine de formule générale (Va), (VIa) ou (VIIa) 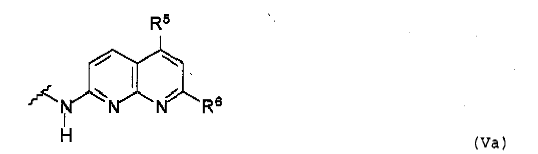 ou 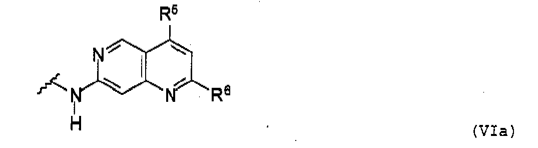 ou 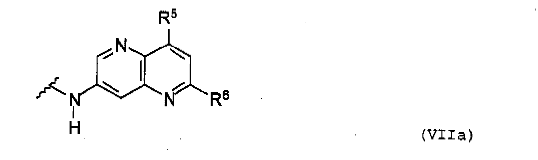 R⁵ ou R⁶ étant indépendamment hydrogène, un groupement alkyle en C₁-C₆, un groupement hydroxyle, un groupement alcoxy en C₁-C₆, un halogène ou NR¹⁰R¹¹, R¹⁰ et R¹¹ étant indépendamment hydrogène ou un groupement alkyle en C₁-C₆.

3. Composé selon la revendication 1 ou 2, **caractérisé en ce que** A est un dérivé phényle de formule générale II et chaque R¹, R² et R³ sont indépendamment un groupement alkyle choisi dans le groupe de méthyle, éthyle, propyle ou butyle.

4. Composé selon la revendication 3, **caractérisé en ce que** chaque R¹, R² et R³ sont un groupement méthyle ou éthyle.

5. A composé selon la revendication 1 ou 2, **caractérisé en ce que** A est le dérivé biphényle de formule générale III et chaque R⁷ est indépendamment hydrogène, ou des groupements méthyle, éthyle, propyle, méthoxy ou éthoxy.

6. Composé selon la revendication 1 ou 2, **caractérisé en ce que** A est un dérivé diphénylalcane de formule générale IV et chaque R⁸ est indépendamment hydrogène, ou des groupements méthyle, éthyle, propyle, hydroxyle, méthoxy ou éthoxy, en particulier des groupements méthyle.

7. Composé selon l'une quelconque des revendications 1, 3 à 6, **caractérisé en ce que** chaque R⁴ est indépendamment hydrogène, ou un groupement méthyle, éthyle, propyle, isopropyle ou butyle.

8. Composé selon l'une quelconque des revendications 1 à 6, **caractérisé en ce que** R⁵ et R⁶ sont indépendamment hydrogène ou un groupement méthyle, éthyle, propyle, isopropyle ou butyle.

9. Composé selon la revendication 8, **caractérisé en ce que** R⁶ est méthyle et R⁵ est hydrogène ou méthyle.

10. Composé selon l'une quelconque des revendications précédentes, **caractérisé en ce que** A est un dérivé phényle de formule générale II et n est 3.

11. Composé selon la revendication 1, **caractérisé en ce que** A est un dérivé biphényle de formule générale III ou un dérivé diphénylalcane de formule générale IV, et **en ce que** n est 4.

12. Composé selon l'une quelconque des revendications précédentes, **caractérisé en ce qu'**il s'agit d'un sel d'halogène.

13. Composition pharmaceutique comprenant un composé selon l'une quelconque des revendications 1 à 12 et éventuellement, un support ou un excipient pharmaceutiquement acceptable.

14. Composé selon l'une quelconque des revendications 1 à 12 ou composés de formule générale (I), compris les stéréoisomères et les sels et solvates de ceux-ci
A-(-CH₂ - B)n (I)
dans laquelle
A est
i) un dérivé phényle de formule générale (II) 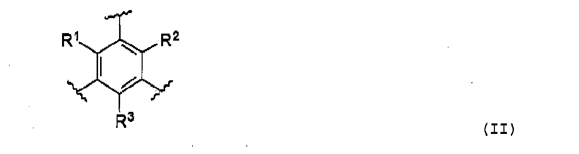 chacun de R¹, R² et R³ étant indépendamment hydrogène ou un groupement alkyle en C₁ - C₆, un groupement alcoxy en C₁ - C₆, un groupement hydroxyle ou un halogène ;
à condition qu'au moins l'un de R¹, R² ou R³ ne soit pas hydrogène,
ii) un dérivé biphényle de formule générale (III) 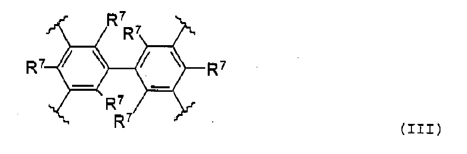 chaque R⁷ étant indépendamment un hydrogène ou un groupement alkyle en C₁ - C₆, un groupement alcoxy en C₁ - C₆, un groupement hydroxyle ou un halogène :
iii) un dérivé diphénylalcane de formule générale (IV) 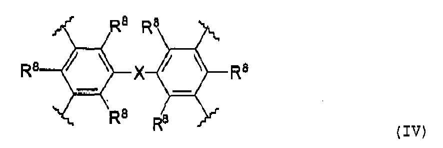 chaque R⁸ étant indépendamment un hydrogène ou un groupement alkyle en C₁ - C₆, un groupement alcoxy en C₁ - C₆, a groupement hydroxyle ou un halogène ; X étant un groupement alkylène en C₁-C₆, en particulier un groupement méthylène ;
B est indépendamment un groupement amino-naphtyridine de formule générale (V), (VI) ou (VII) 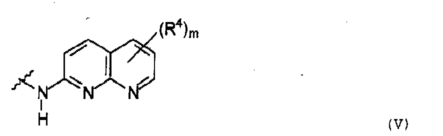 ou 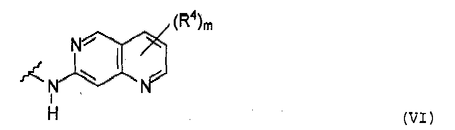 ou 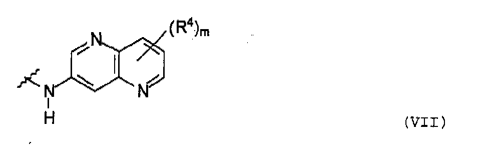 chaque R⁴ étant indépendamment un hydrogène, un groupement alkyle en C₁-C₆, un groupement hydroxyle, un groupement alcoxy en C₁-C₆, un halogène ou NR¹⁰R¹¹, R¹⁰ et R¹¹ étant indépendamment hydrogène, ou un groupement alkyle en C₁-C₆, et m étant un entier de 2 à 5,
et n est un entier de 1, 2 ou 3 si A est un dérivé phényle de formule (II) ou n est 1, 2, 3 ou 4 si A est (III) ou (IV), et dans laquelle lorsque n est 1 ou 2, si A est un dérivé phényle ou lorsque n est 1, 2 ou 3, si A est un dérivé biphényle ou un dérivé diphénylalcane, le motif espaceur est substitué en outre par l'un quelconque des substituants suivants :
- CH₂-Y où Y est 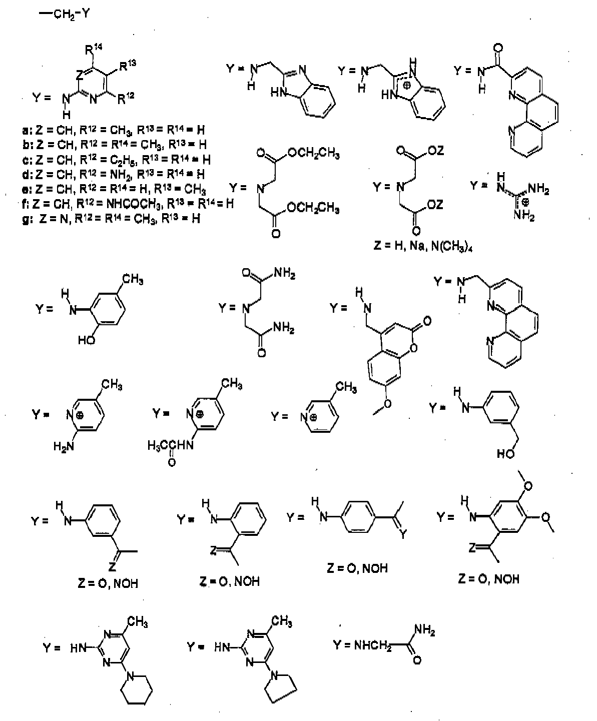
ou hydrogène, un groupement alkyle en C₁-C₆, un groupement alcoxy en C₁-C₆, un groupement hydroxyle ou un halogène, de préférence hydrogène, un groupement méthyle, éthyle, propyle ou hydroxyle, destiné à être utilisé pour la prévention ou le traitement d'une infection ou du cancer.
